# EUROPEAN PATENT APPLICATION

(11) **EP 2 749 561 A1**
(43) Date of publication of application: **02.07.2014**
(21) Application number: 13195602.1
(22) Date of filing: 04.12.2013
(51) Int. Cl.: C07D 471/04, A61K 31/4745

(54) **Radioactive quinolinone derivative and pharmaceutical drug comprising the same**

(30) Priority: 27.12.2012 JP 2012289454
(71) Applicant: Kyoto University, Kyoto-shi, Kyoto 606-8501 (JP); NIHON MEDI-PHYSICS CO., LTD., Tokyo 136-0075 (JP)
(72) Inventor: Saji, Hideo, Kyoto-shi,, Kyoto 606-8501 (JP); Kimura, Hiroyuki, Kyoto-shi,, Kyoto 606-8501 (JP); Ono, Masahiro, Kyoto-shi,, Kyoto 606-8501 (JP); Matsumoto, Hiroki, Sodegaura City,, Chiba 299-0266 (JP)
(74) Representative: TBK

(57) **Abstract**

It is intended to provide a radioactive compound represented by the following general formula (1) or a salt thereof that has higher selectivity for CYP11B2 than that for CYP11B1, exhibits highly selective accumulation in the adrenal gland compared with blood and organs adjacent to the adrenal gland, and permits commercial supply.

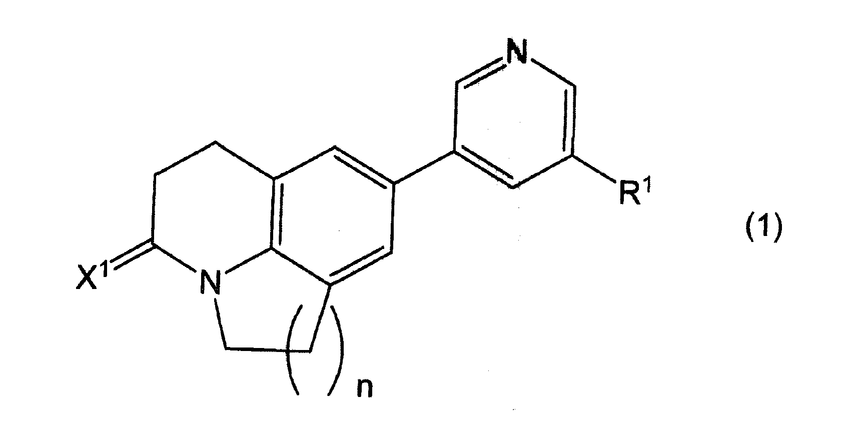

## Description

This application is based on Japanese patent application NO. 2012-289454, the content of which are incorporated hereinto by reference.

### BACKGROUND

### TECHNICAL FIELD

The present invention relates to a radioactive quinolinone derivative and a pharmaceutical drug comprising the same.

### RELATED ART

Primary aldosteronism (PA) is known as a disease that is developed by an abnormality of the adrenal gland. This disease primary aldosteronism begins with a tumor in the adrenal cortex, which in turn overproduces aldosterone, leading to hypertension or hypokalemia. The primary aldosteronism often forms a tumor in either right or left adrenal gland. The tumor formed on one side can be treated with surgery. When tumors have been formed on both sides, treatment with medication is adopted for this disease. Thus, localization to determine whether a tumor(s) in the developed primary aldosteronism resides on one side or on both sides is necessary for deciding the course of treatment.

Computed tomography (CT), adrenal scintigraphy using adosterol, or adrenal venous sampling (AVS) is used as this localization.

CT, however, is incapable of functional diagnosis in principle. This approach is therefore unable to differentiate non-functioning adenoma and aldosterone-producing adenoma even if a lesion is observed in the adrenal gland.

Alternatively, the adrenal scintigraphy using adosterol is complicated in such a way that this approach requires pretreatment with dexamethasone and a long examination period. In addition, the accumulation of adosterol in adrenal scintigraphy has been shown in recent years to depend on the size of adrenal adenoma rather than the ability to produce aldosterone. The diagnostic approach using adosterol reportedly localizes PA with very low accuracy (Nomura K, et al., The Journal of Clinical Endocrinology & Metabolism, 1990, Vol. 71, p. 825-830).

At the time of filing the present application, AVS is allegedly a gold standard for confirming the position of a lesion oversecreting aldosterone (Nishikawa T, et al., Endocrine Journal, 2011, Vol. 58, No. 9, p. 711-721). AVS, however, involves collecting blood by adrenal vein catheterization and places a significant burden on patients, for example, due to necessary hospitalization or anesthesia. Thus, highly skilled physicians are required.

Thus, attempts to image adrenal adenoma by single-photon emission computed tomography (SPECT) or positron emission tomography (PET) have been made in recent years with the aim of achieving the noninvasive localization of aldosterone-producing adenoma as a substitute for AVS. International Publication Nos. WO 2007/144725 and WO 2011/151411; Georg Zettinig, et al., European Journal of Nuclear Medicine and Molecular Imaging, 2004, Vol. 31, No. 9, p. 1224-1230; Wolfgang Wadsak, et al., European Journal of Nuclear Medicine and Molecular Imaging, 2006, Vol. 33, No. 6, p. 669-672; Stefanie Hahner, et al., Journal of Clinical Endocrinology & Metabolism, 2008, Vol. 93, No. 6, p. 2358-2365; and Timothy J. Burton, et al., Journal of Clinical Endocrinology & Metabolism, 2012, Vol. 97, No. 1, p. 100-109 have reported various radioactive compounds targeting a steroid biosynthetic enzyme (CYPB1 or CYPB2). For example, Georg Zettinig, et al., European Journal of Nuclear Medicine and Molecular Imaging, 2004, Vol. 31, No. 9, p. 1224-1230; and Timothy J. Burton, et al., Journal of Clinical Endocrinology & Metabolism, 2012, Vol. 97, No. 1, p. 100-109 describe ¹¹C-labeled metomidate. Wolfgang Wadsak, et al., European Journal of Nuclear Medicine and Molecular Imaging, 2006, Vol. 33, No. 6, p. 669-672; and Stefanie Hahner, et al., Journal of Clinical Endocrinology & Metabolism, 2008, Vol. 93, No. 6, p. 2358-2365 describe ¹⁸F-labeled etomidate. Stefanie Hahner, et al., Journal of Clinical Endocrinology & Metabolism, 2008, Vol. 93, No. 6, p. 2358-2365 describes ¹²³I-labeled iodometomidate.

### SUMMARY

In the steroid biosynthesis system, aldosterone synthase (CYP11B2) is an enzyme involved only in aldosterone synthesis, whereas steroid 11β-hydroxylase (CYP11B1) is involved in the synthesis of both aldosterone and cortisol. Thus, high selectivity for CYP11B2 is preferred for detecting the oversecretion of aldosterone in distinction from the oversecretion of cortisol.

Nevertheless, International Publication No. WO 2007/144725 describes a compound targeting CYP11B1. Also, Stefanie Hahner, et al., Journal of Clinical Endocrinology & Metabolism, 2008, Vol. 93, No. 6, p. 2358-2365 has reported that metomidate and etomidate have higher selectivity for steroid 11β-hydroxylase (CYP11B1) than that for aldosterone synthase (CYP11B2). Thus, the techniques of International Publication No. WO 2007/144725; Georg Zettinig, et al., European Journal of Nuclear Medicine and Molecular Imaging, 2004, Vol. 31, No. 9, p. 1224-1230; Wolfgang Wadsak, et al., European Journal of Nuclear Medicine and Molecular Imaging, 2006, Vol. 33, No. 6, p. 669-672; Stefanie Hahner, et al., Journal of Clinical Endocrinology & Metabolism, 2008, Vol. 93, No. 6, p. 2358-2365; and Timothy J. Burton, et al., Journal of Clinical Endocrinology & Metabolism, 2012, Vol. 97, No. 1, p. 100-109 have difficulty in detecting the production of aldosterone in distinction from the production of cortisol.

By contrast, International Publication No. WO 2011/151411 describes an ¹⁸F-labeled compound with higher selectivity for CYP11B2 than that for CYP11B1. Meanwhile, the noninvasive imaging of adrenal adenoma by SPECT or PET requires higher accumulation in the adrenal gland than that in blood or organs adjacent to the adrenal gland (e.g., kidney). International Publication No. WO 2011/151411, however, discloses no example in which the *in vivo* distribution of the ¹⁸F-labeled compound was confirmed. The technique described in International Publication No. WO 2011/151411 therefore does not show whether or not the compound can selectively accumulate in the adrenal gland compared with organs adjacent to the adrenal gland.

The ¹¹C-labeled metomidate disclosed in Georg Zettinig, et al., European Journal of Nuclear Medicine and Molecular Imaging, 2004, Vol. 31, No. 9, p. 1224-1230; and Timothy J. Burton, et al., Journal of Clinical Endocrinology & Metabolism, 2012, Vol. 97, No. 1, p. 100-109 exhibits an ¹¹C half-life as short as 20 minutes and is thus difficult to commercially supply.

The present invention has been made in light of these situations, and an object of the present invention is to provide a radioactive compound that has higher selectivity for CYP11B2 than that for CYP11B1, exhibits highly selective accumulation in the adrenal gland compared with blood and organs adjacent to the adrenal gland, and permits commercial supply.

Specifically, the present invention provides a radioactive compound represented by the following general formula (1) or a salt thereof:

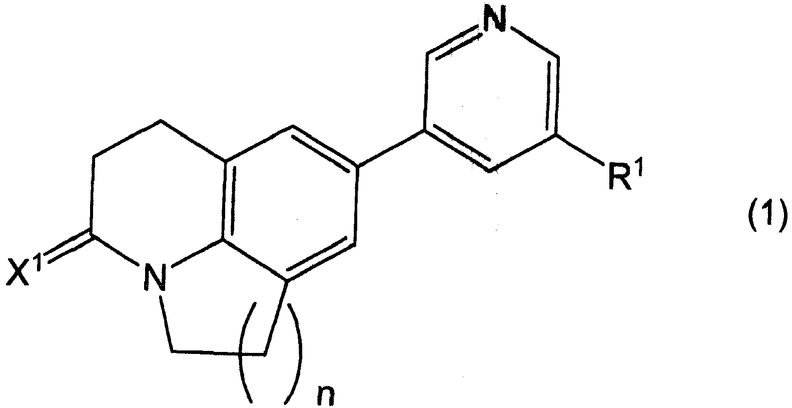

wherein R¹ represents -X² or -O(CH₂)ₘ-X²; X¹ represents an oxygen atom or a sulfur atom; X² represents a radiohalogen atom; n represents an integer of 1 or 2; and m represents an integer of 1 to 3.

The present invention provides a radioactive compound that has higher selectivity for CYP11B2 than that for CYP11B1, exhibits highly selective accumulation in the adrenal gland compared with blood and organs adjacent to the adrenal gland, and permits commercial supply.

### BRIEF DESCRIPTION OF THE DRAWINGS

The above and other objects, advantages and features of the present invention will be more apparent from the following description of certain preferred embodiments taken in conjunction with the accompanying drawing.
FIG. 1 is a diagram showing a synthesis example of a labeling precursor and an unlabeled form of 9-(5-[¹²⁵I]iodopyridin-3-yl)-1,2,6,7-tetrahydro-5H-pyrido[3,2,1-ij]quinolin-3-one;
FIG. 2 is a diagram showing a labeling and synthesis example of 9-(5-[¹²⁵I]iodopyridin-3-yl)-1,2,6,7-tetrahydro-5H-pyrido[3,2,1-ij]quinolin-3-one and 8-(5-[¹²⁵I]iodopyridin-3-yl)-1,2,5,6-tetrahydro-4H-pyrrolo[3,2,1-ij]quinolin-4-one;
FIGs. 3A and 3B are diagrams showing the HPLC analysis results about the radiochemical purity of a radioiodine-labeled form. FIG. 3A shows the results about 9-(5-[¹²⁵I]iodopyridin-3-yl)-1,2,6,7-tetrahydro-5H-pyrido[3,2,1-ij]quinolin-3-one. FIG. 3B shows the results about 8- (5- [¹²⁵I]iodopyridin-3-yl)-1,2,5,6-tetrahydro-4H-pyrrolo[3,2,1-ij]quinolin-4-one;
FIG. 4 is a diagram showing time-dependent change in the pharmacokinetic and distribution of 9-(5-[¹²⁵I]iodopyridin-3-yl)-1,2,6,7-tetrahydro-5H-pyrido[3,2,1-ij]quinolin-3-one;
FIG. 5 is a diagram showing a synthesis example of a labeling precursor and an unlabeled form of 8-(5-[¹²⁵I]iodopyridin-3-yl)-1,2,5,6-tetrahydro-4H-pyrrolo[3,2,1-ij]quinolin-4-one;
FIG. 6 is a diagram showing time-dependent change in the pharmacokinetic and distribution of 8-(5-[¹²⁵I]iodopyridin-3-yl)-1,2,5,6-tetrahydro-4H-pyrrolo[3,2,1-ij]quinolin-4-one;
FIG. 7 is a diagram showing the evaluation results about the stability of 8-(5-[¹²⁵I]iodopyridin-3-yl)-1,2,5,6-tetrahydro-4H-pyrrolo[3,2,1-ij]quinolin-4-one in plasma;
FIG. 8 is a diagram showing a synthesis example of a labeling precursor of 9-(5-(2-[¹⁸F]fluoroethyloxy)pyridin-3-yl)-1,2,6,7-tetrahydro-5H-pyrido[3,2,1-ij]quinolin-3-one;
FIG. 9 is a diagram showing a synthesis example of an unlabeled form of 9-(5-(2-fluoroethyloxy)pyridin-3-yl)-1,2,6,7-tetrahydro-5H-pyrido[3,2,1-ij]quinolin-3-one;
FIG. 10 is a diagram showing a labeling and synthesis example of 9-(5-(2-[¹⁸F]fluoroethyloxy)pyridin-3-yl)-1,2,6,7-tetrahydro-5H-pyrido[3,2,1-ij]quinolin-3-one;
FIG. 11 is a diagram showing time-dependent change in the pharmacokinetic and distribution of 9-(5-(2-fluoroethyloxy)pyridin-3-yl)-1,2,6,7-tetrahydro-5H-pyrido[3,2,1-ij]quinolin-3-one;
FIG. 12 is a diagram showing a synthesis example of a labeling precursor of 8-(5-(2-[¹⁸F]fluoroethyloxy)pyridin-3-yl)-1,2,5,6-tetrahydro-4H-pyrrolo[3,2,1-ij]quinolin-4-one;
FIG. 13 is a diagram showing a synthesis example of an unlabeled form of 8-(5-(2-[¹⁸F]fluoroethyloxy)pyridin-3-yl)-1,2,5,6-tetrahydro-4H-pyrrolo[3,2,1-ij]quinolin-4-one;
FIG. 14 is a diagram showing a labeling and synthesis example of 8- (5- (2- [¹⁸F]fluoroethyloxy)pyridin-3-yl)-1,2,5,6-tetrahydro-4H-pyrrolo[3,2,1-ij]quinolin-4-one;
FIG. 15 is a diagram showing time-dependent change in the pharmacokinetic and distribution of 8-(5-(2-[¹⁸F]fluoroethyloxy)pyridin-3-yl)-1,2,5,6-tetrahydro-4H-pyrrolo[3,2,1-ij]quinolin-4-one;
FIGs. 16A to 16F are diagrams showing the evaluation results about affinity and selectivity for CYP11B1 and CYP11B2. FIGS. 16A, 16C, and 16E show the inhibition curves against CYP11B2. FIGS. 16B, 16D, and 16F show the inhibition curves against CYP11B1; and
FIGs. 17A to D are diagrams showing the evaluation results about affinity and selectivity for CYP11B1 and CYP11B2. FIGS. 17A and 17C show the inhibition curves against CYP11B2. FIGS. 17B and 17D show the inhibition curves against CYP11B1.

### DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposed.

In the present invention, the "radiohalogen atom" refers to at least one atom selected from fluorine, chlorine, bromine, and iodine radioisotopes. Preferably ¹⁸F, ¹²³I, ¹²⁴I, ¹²⁵I, ¹³¹I, or ⁷⁶Br can be used.

In the present invention, a radioactive compound represented by the general formula (1) wherein X¹ represents an oxygen atom, or a salt thereof is preferred from the viewpoint of affinity for CYP11B2.

In the present invention, the "salt" can be any pharmaceutically acceptable salt.

The radioactive compound represented by the general formula (1) may be a base. In such a case, the compound can be made into a salt induced from, for example, an inorganic acid such as hydrochloric acid, hydrobromic acid, sulfuric acid, nitric acid, or phosphoric acid, or an organic acid such as acetic acid, maleic acid, succinic acid, mandelic acid, fumaric acid, malonic acid, pyruvic acid, oxalic acid, glycolic acid, salicylic acid, pyranosidyl acid (glucuronic acid, galacturonic acid, etc.), α-hydroxy acid (citric acid, tartaric acid, etc.), amino acid (aspartic acid, glutamic acid, etc.), aromatic acid (benzoic acid, cinnamic acid, etc.), or sulfonic acid (p-toluenesulfonic acid, ethanesulfonic acid, etc.).

Alternatively, the radioactive compound of the present invention represented by the general formula (1) may be an acid. In such a case, the compound can be made into a salt induced from, for example, an organic base such as amino acid (glycine, arginine, etc.), ammonia, primary, secondary, or tertiary amine, or cyclic amine (piperidine, morpholine, piperazine, etc.), or an inorganic base such as sodium hydroxide, calcium hydroxide, potassium hydroxide, magnesium hydroxide, manganese hydroxide, iron hydroxide, copper hydroxide, zinc hydroxide, aluminum hydroxide, or lithium hydroxide.

A radioactive compound represented by the general formula (1) wherein R¹ is a radiohalogen atom is represented by the following general formula (2):

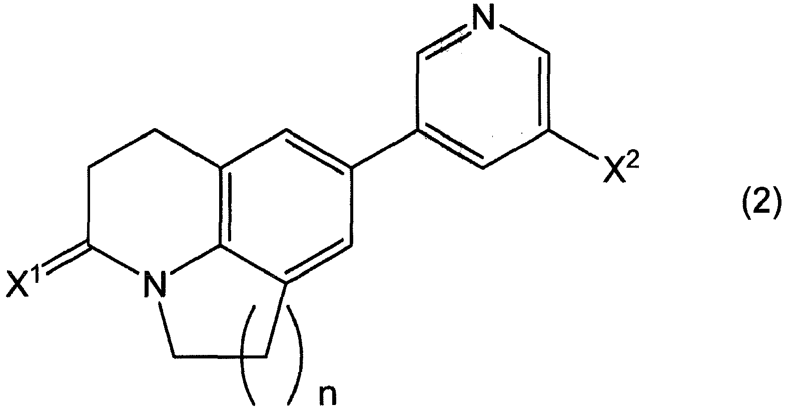

wherein X¹ represents an oxygen atom or a sulfur atom; X² represents a radiohalogen atom; and n represents an integer of 1 or 2.

In the general formula (2), X¹ is preferably an oxygen atom, and X² is preferably a radioiodine atom. The radioiodine atom is preferably ¹²³I, ¹²⁴I, ¹²⁵I, or 131I. n is preferably an integer of 2 from the viewpoint of highly selective accumulation in the adrenal gland compared with blood and organs adjacent to the adrenal gland.

Alternatively, a compound represented by the general formula (1) wherein R¹ is -O(CH₂)ₘ-X² is represented by the following general formula (3):

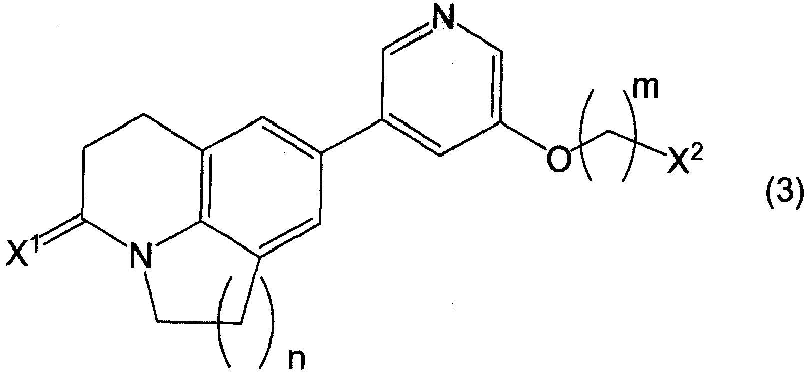

wherein X¹ represents an oxygen atom or a sulfur atom; X² represents a radiohalogen atom; n represents an integer of 1 or 2; and m represents an integer of 1 to 3.

X¹ is preferably an oxygen atom, and X² is preferably a radiofluorine atom. m is preferably an integer of 2 or 3, more preferably an integer of 2. n is preferably an integer of 2 from the viewpoint of highly selective accumulation in the adrenal gland compared with blood and organs adjacent to the adrenal gland.

Among the radioactive compounds represented by the general formulas (2) and (3), the compound represented by the general formula (3) is more preferred from the viewpoint of stability.

Hereinafter, a method for producing the radioactive compound of the present invention will be described by taking one aspect of a method for producing each radioactive compound represented by the general formula (2) or (3) as an example. Scheme 1 shows one example of the synthesis route of a labeling precursor of the radioactive compound represented by the general formula(2).

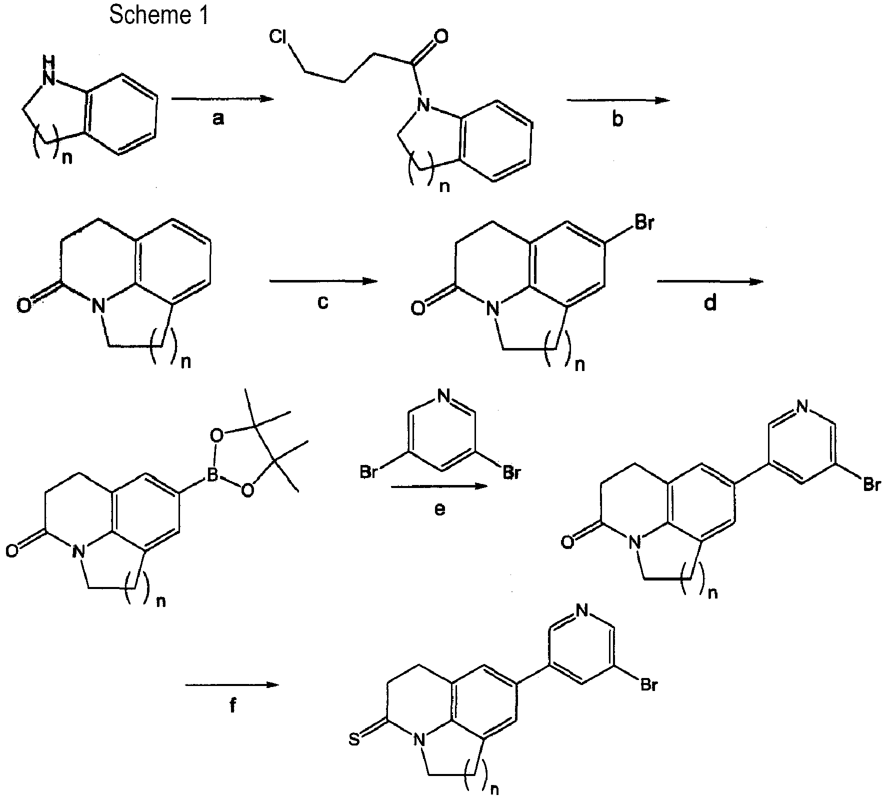

A starting material indoline (n = 1) or tetrahydroquinoline (n = 2) is first acylated using an acetone solution of ω-chlorocarboxylic acid chloride (Step a) and subsequently subjected to intramolecular Friedel-Crafts alkylation in a molten AlCl₃/NaCl mixture at 150°C (Step b). A para-substituted product is selectively produced by bromination using a DMF solution of NBS at 0°C (Step c). This product is converted to boronic acid ester through reaction with bis(pinacolato)diboron in the presence of a palladium catalyst (Step d).

Then, an aqueous sodium carbonate solution and tetrakis(triphenylphosphine)palladium(0) are added into a toluene-ethanol mixed solution of the boronic acid ester and 3,5-dibromopyridine, and the mixture is stirred under heating to reflux to perform Suzuki coupling (Step e). The obtained oxo compound can be derivatized into a corresponding thio analog using a Lawesson's reagent (Step f). In this way, a compound represented by the following general formula (5) can be obtained:

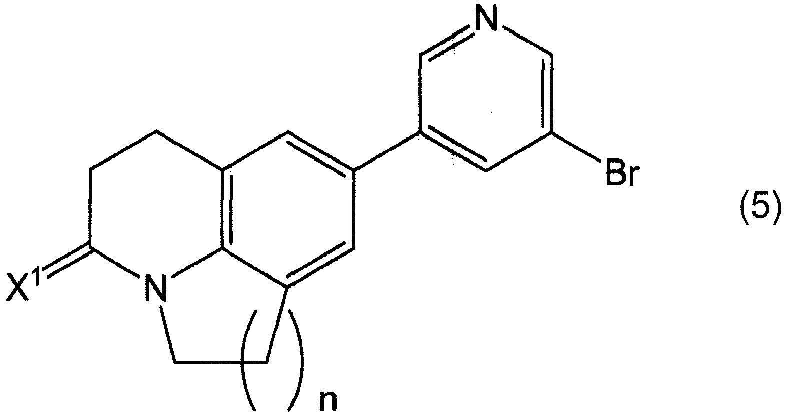

wherein X¹ represents an oxygen atom or a sulfur atom; and n represents an integer of 1 or 2.

The compound represented by the general formula (5) or a salt thereof can be subjected to halogen exchange with radiohalide ions to obtain a compound represented by the general formula (2). For example, a compound represented by the general formula (2) wherein X² represents ¹⁸F is obtained using ¹⁸F-fluoride ions. Alternatively, a compound represented by the general formula (2) wherein X² represents ⁷⁶Br is obtained using ⁷⁶Br-bromide ions.

In the case of a compound represented by the general formula (2) wherein X² is a radioiodine atom, the compound represented by the general formula (5) can be trialkylstannylated or trialkylsilylated to obtain a compound represented by the following general formula (6) as a labeling precursor:

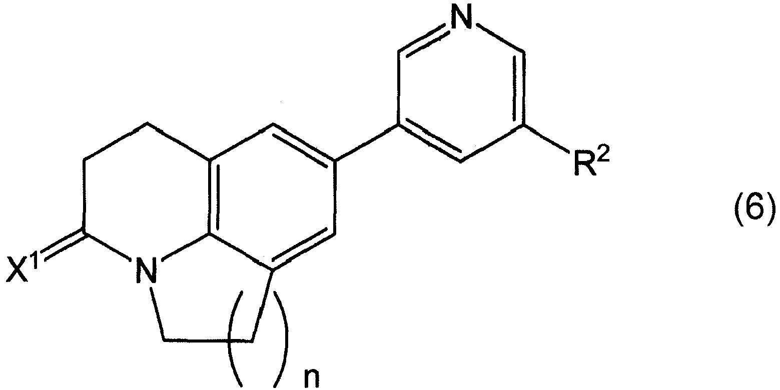

wherein X¹ represents an oxygen atom or a sulfur atom; R² represents a trialkyltin group or a trialkylsilyl group; and n represents an integer of 1 or 2.

Examples of the trialkyltin group represented by R² in the general formula (6) include tri(C1-C6 alkyl)tin groups. A tributyltin group is more preferred. Examples of the trialkylsilyl group include tri(C1-C6 alkyl)silyl groups. A trimethylsilyl group is more preferred.

The compound represented by the general formula (6) can be subjected to radioiodination reaction to obtain a compound represented by the general formula (2) wherein X² is a radioiodine atom. The radioiodination reaction can be performed through the reaction between alkali metal radioiodide and an oxidizing agent under acidic conditions. Examples of the alkali metal radioiodide that can be used include sodium compounds of radioiodine and potassium compounds of radioiodine. Examples of the oxidizing agent that can be used include chloramine-T, hydrogen peroxide water, peracetic acid, N-chlorosuccinimide, and N-bromosuccinimide.

Next, one aspect of a method for producing the radioactive compound represented by the general formula (3) will be described. Schemes 2 and 3 show one example of the synthesis route of a labeling precursor of the radioactive compound represented by the general formula (3). First, boronic acid ester is synthesized from indoline (n = 1) or tetrahydroquinoline (n = 2) through Steps a to d according to the method of Scheme 1.

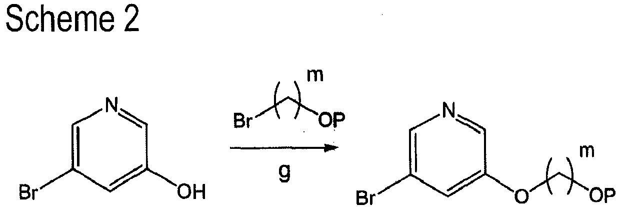

According to Scheme 2, ω-bromoalkanol (bromomethanol (m = 1), 2-bromoethanol (m = 2), or 3-bromopropanol (m = 3)) with a protected hydroxy group is reacted with 3-bromo-5-hydroxypyridine in the presence of potassium carbonate in a DMF solution (Step g).

The hydroxy group of the ω-bromoalkanol may be protected by a non-limiting method that can be used in alcohol protection, and can be protected by a method described in, for example, Greene's Protective Groups in Organic Synthesis, p. 17-245 (Wiley-Interscience; 4th edition). For example, a trityl, monomethoxytrityl, dimethoxytrityl, trimethoxytrityl, methoxymethyl, 1-ethoxyethyl, methoxyethoxymethyl, benzyl, p-methoxybenzyl, 2-tetrahydropyranyl, trimethylsilyl, triethylsilyl, t-butyldimethylsilyl, t-butyldiphenylsilyl, acetyl, propanoyl, pivaloyl, palmitoyl, dimethylaminomethylcarbonyl, alanyl, 2,2,2-trichloroethoxycarbonyl, benzoyl, or allyloxycarbonyl group can be preferably used as a protective group (P in Scheme 2) for the hydroxy group of the ω-bromoalkanol.

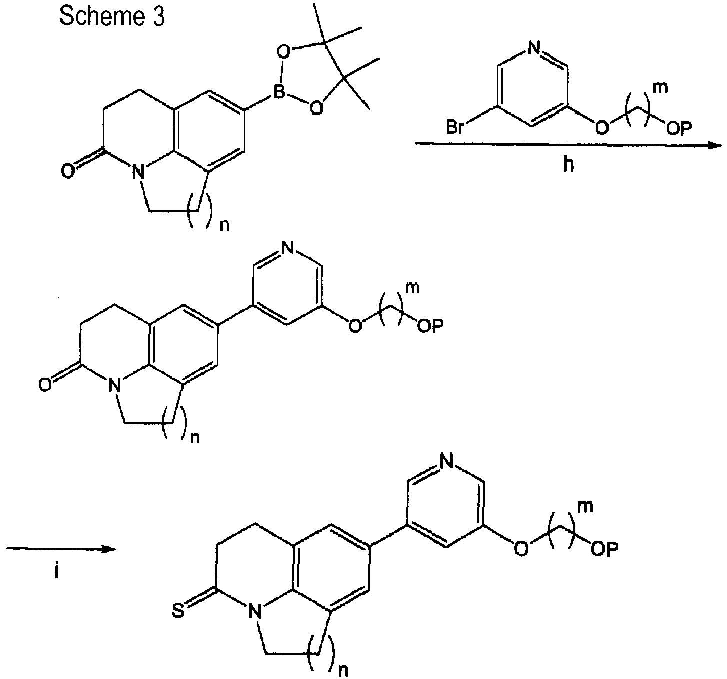

The boronic acid ester obtained in Scheme 1 and the pyridine derivative obtained in Scheme 2 are dissolved in a toluene-ethanol mixed solution. To this solution, an aqueous sodium carbonate solution and tetrakis(triphenylphosphine)palladium(0) are added, and the mixture is stirred under heating to reflux to perform Suzuki coupling (Step h). The obtained oxo compound can be derivatized into a corresponding thio analog using a Lawesson's reagent (Step i). In this way, a compound represented by the following general formula (7) can be obtained:

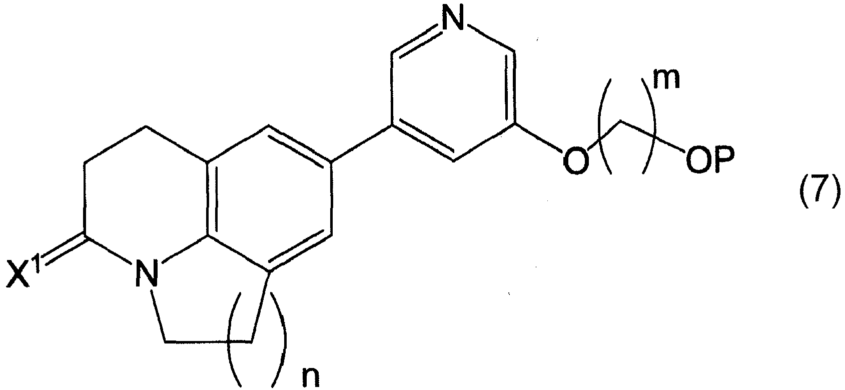

wherein X¹ represents an oxygen atom or a sulfur atom; P represents a protective group for the alcohol; n represents an integer of 1 or 2; and m represents an integer of 1 to 3.

After deprotection of the protective group in the alcohol of the compound represented by the general formula (7), the resulting compound can be halogenated or sulfonylated to obtain a compound represented by the following general formula (8) as a labeling precursor:

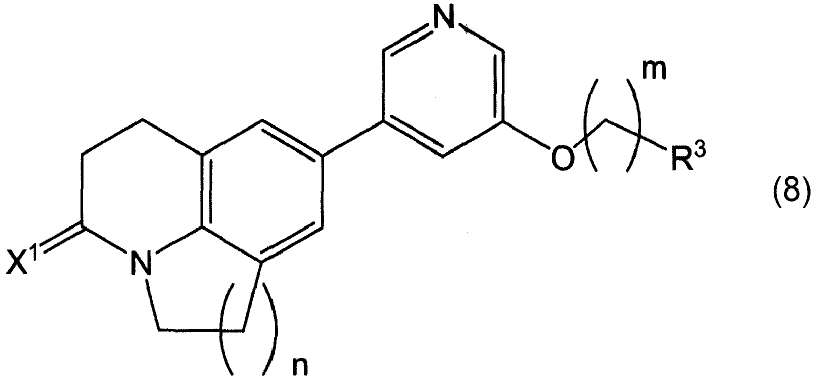

wherein X¹ represents an oxygen atom or a sulfur atom; R³ represents a halogen atom, a substituted or unsubstituted alkylsulfonyloxy group, or a substituted or unsubstituted arylsulfonyloxy group; n represents an integer of 1 or 2; and m represents an integer of 1 to 3.

When R³ in the general formula (8) is a halogen atom, a bromine atom or an iodine atom is preferred. The substituted or unsubstituted alkylsulfonyloxy group is preferably an alkylsulfonyloxy group having 1 to 12 carbon atoms. The substituted alkylsulfonyloxy group may have a hydrogen atom replaced by a halogen atom. The substituted or unsubstituted arylsulfonyloxy group is preferably a substituted or unsubstituted benzenesulfonyloxy group, more preferably a substituted benzenesulfonyloxy group, further preferably a benzenesulfonyloxy group substituted by an alkyl group having 1 to 12 carbon atoms or a nitro group. Preferred examples of R³ in the general formula (8) include methanesulfonyloxy, benzenesulfonyloxy, p-toluenesulfonyloxy, p-nitrobenzenesulfonyloxy, and trifluoromethanesulfonyloxy groups.

The compound represented by the general formula (8) can be reacted with radiohalide ions to obtain a compound represented by the general formula (3). For example, a compound of the general formula (3) wherein X² represents a radiofluorine atom can be obtained using radiofluoride ions. The radiofluorination reaction is preferably performed in the presence of a base and may be performed in the presence of any of various phase transfer catalysts such as 4,7,13,16,21,24-hexaoxa-1,10-diazabicyclo[8.8.8]-hexacosane (trade name: Kryptofix 222).

When the radioactive compound represented by the general formula (2) is produced with the compound represented by the general formula (5) or (6) as a labeling precursor or when the radioactive compound represented by the general formula (3) is produced with the compound represented by the general formula (8) as a labeling precursor, these compounds represented by the general formulas (5), (6), and (8) may each form a salt. Examples of this salt include the same as those exemplified as the salt of the radioactive compound represented by the general formula (1).

The radioactive compound represented by the general formula (1) or the salt thereof may be used in a pharmaceutical drug. In such a case, unreacted radiohalogen ions and insoluble impurities are desirably removed by purification using a membrane filter, a column packed with any of various fillers, HPLC, or the like.

In the present invention, a pharmaceutical drug can also be prepared from the radioactive compound or the salt thereof thus produced. The "pharmaceutical drug" described herein can be defined as a formulation comprising the radioactive compound represented by the general formula (1) or the salt thereof in a form suitable for administration into a living body. This pharmaceutical drug is preferably administered through a parenteral route, i.e., through injection. The pharmaceutical drug is more preferably in the dosage form of an aqueous solution. Such a composition may additionally comprise appropriate optional ingredients such as a pH adjuster, a pharmaceutically acceptable solubilizer, a stabilizer, and/or an antioxidant.

Upon introduction of the pharmaceutical drug according to the present invention into a living body, the radioactive compound represented by the general formula (1) accumulates in an aldosterone- producing region. This region can therefore be imaged by the noninvasive detection of the radiation from outside the living body using a radioactivity detector, a single-photon emission computed tomography scanner, a positron emission tomography scanner, autoradiography, or the like. As a result, increase or decrease in aldosterone production can be diagnosed. Thus, the pharmaceutical drug of the present invention can be used as a diagnostic imaging agent and, specifically, can be applied to a diagnostic imaging agent for positron emission tomography or a diagnostic imaging agent for single-photon emission computed tomography. For example, in the case of using a positron-emitting radionuclide such as ¹⁸F, ⁷⁶Br, or ¹²⁴I as the radiohalogen atom, the pharmaceutical agent can be used as a diagnostic imaging agent for positron emission tomography. In the case of using ¹²³I as the radiohalogen atom, the pharmaceutical agent can be used as a diagnostic imaging agent for single-photon emission computed tomography. Preferably, the diagnostic imaging agent according to the present invention can be used in the diagnostic imaging of adrenal disease (aldosterone-producing tumor, etc.) caused by aldosterone overproduction.

### Examples

Hereinafter, the present invention will be described more specifically with reference to Examples. However, the present invention is not limited by the contents of these Examples. In Examples below, the name of each compound subjected to an experiment is defined as follows:
PAY15: 9-(5-iodopyridin-3-yl)-1,2,6,7-tetrahydro-5H-pyrido[3,2,1-ij]quinolin-3-one
PAY21: 8-(5-iodopyridin-3-yl)-1,2,5,6-tetrahydro-4H-pyrrolo[3,2,1-ij]quinolin-4-one
PAY27: 9-(5-(2-fluoroethyloxy)pyridin-3-yl)-1,2,6,7-tetrahydro-5H-pyrido[3,2,1-ij]quinolin-3-one
PAY54: 8-(5-(2-fluoroethyloxy)pyridin-3-yl)-1,2,5,6-tetrahydro-4H-pyrrolo[3,2,1-ij]quinolin-4-one

In Examples, the molecular structure of each compound based on an NMR spectrum was identified by a ¹H-NMR spectrum. The ¹H-NMR spectrum was obtained using JNM-AL400 NMR spectrometer (JEOL Ltd.) as an NMR apparatus. The resonant frequency was set to 400 MHz. The solvent used was deuterated chloroform. The signal δ7.26 of the deuterated chloroform was used as a reference. All chemical shifts were indicated by ppm on the data scale (δ). The fine splitting of signals was indicated by abbreviations (s: singlet, d: doublet, t: triplet, dd: double doublet, dt: double triplet, td: triple doublet, m: multiplet, brs: broad signal).

### Example 1: Synthesis of labeling precursor of [¹²⁵I]PAY15

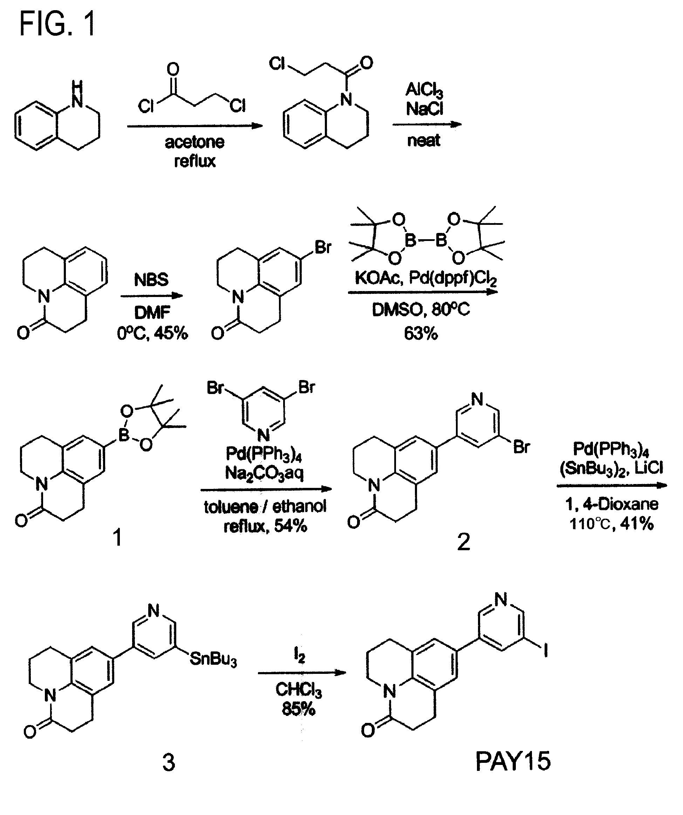

A labeling precursor of [¹²⁵I]PAY15 was synthesized according to the scheme shown in FIG. 1.

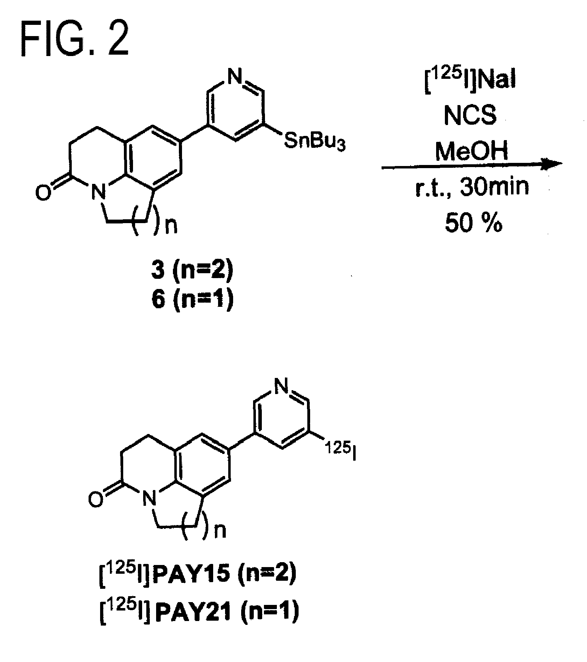

In FIGS. 1 and 2, the name of each compound is as follows:
Compound 1: 9-boronic acid pinacol ester-1,2,6,7-tetrahydro-5H-pyrido[3,2,1-ij]quinolin-3-one
Compound 2: 9-(5-bromopyridin-3-yl)-1,2,6,7-tetrahydro-5H-pyrido[3,2,1-ij]quinolin-3-one
Compound 3: 9-(5-tributylstannylpyridin-3-yl)-1,2,6,7-tetrahydro-5H-pyrido[3,2,1-ij]quinolin-3-one

### 1) Synthesis of compound 1

Compound 1 was synthesized according to a method described in Journal of Medicinal Chemistry, 2011, vol. 54, p. 2307-2319 with 1,2,3,4-tetrahydroquinoline as a starting material.

### 2) Synthesis of compound 2 from compound 1

In an argon atmosphere, a 1 mol/L aqueous sodium carbonate solution (630 µL, 0.63 mmol) and tetrakis(triphenylphosphine)palladium(0) (12 mg, 0.010 mmol) were added in this order to a toluene-ethanol (1.6/0.4 mL) mixed solution of compound 1 (33 mg, 0.10 mmol) and 3,5-dibromopyridine (37 mg, 0.16 mmol) under stirring at room temperature (25°C), and the mixture was stirred at the same temperature as above for 10 minutes and then stirred for 6 hours under heating to reflux. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (ethyl acetate : hexane (volume ratio) = 1 : 1) to obtain compound 2 (22 mg, yield relative to compound 1: 54%) in a colorless solid form.

Compound 2: ¹H-NMR δ: 8.72 (d, J = 1.4 Hz, 1H), 8.61 (d, J = 1.6 Hz, 1H), 7.99 (d, J = 1.4 Hz, 1H), 7.31 (s, 2H), 3.91 (t, J = 5.8 Hz, 2H), 2.96 (t, J = 7.1 Hz, 2H), 2.86 (t, J = 5.8 Hz, 2H), 2.69 (t, J = 7.6 Hz, 2H), 1.99 (t, J=6.0 Hz, 2H)

### 3) Synthesis of compound 3 from compound 2

In an argon atmosphere, bistributyltin (35 µL, 0.07 mmol) and tetrakis(triphenylphosphine)palladium(0) (4.0 mg, 0.003 mmol) were added in this order to a dioxane (2.0 mL) solution of compound 2 (12 mg, 0.035 mmol) and lithium chloride (4.4 mg, 0.10 mmol) under stirring at room temperature, and the mixture was stirred at the same temperature as above for 10 minutes and then stirred at 110°C for 12 hours. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer aminosilica gel column chromatography (ethyl acetate : hexane = 1 : 1) to obtain compound 3 (8.0 mg, yield relative to compound 2: 41%). Similar operation was repeated using the remaining 10 mg of compound 2 obtained in the preceding step 2).

Compound 3: ¹H-NMR δ: 8.69 (d, J = 2.5 Hz, 1H), 8.54 (s, 1H), 7.85 (s, 1H), 7.20 (brs, 2H), 3.92 (t, J = 5.9 Hz, 2H), 2.97 (t, J = 7.3 Hz, 2H), 2.88 (t, J = 6.2 Hz, 2H), 2.70 (t, J = 7.3 Hz, 2H), 1.99 (td, J = 5.9 Hz, 6.2 Hz, 2H), 1.60-1.53 (m, 6H), 1.40-1.30 (m, 6H), 1.17-1.11 (m, 6H), 0.90 (t, J = 7.2 Hz, 9H)

### Example 2: Synthesis of PAY15 (unlabeled form)

An unlabeled form of PAY15 was synthesized according to the scheme shown in FIG. 1.

In a nitrogen atmosphere, iodine (2.0 mg, 0.016 mmol) was added to a chloroform (1.0 mL) solution of compound 3 (8.0 mg, 0.014 mmol) obtained in Example 1 under stirring at room temperature, and the mixture was stirred at the same temperature as above for 30 minutes. Then, a saturated aqueous solution of sodium thiosulfate was added thereto, followed by extraction three times with chloroform. The extract was washed with saturated saline and dried over anhydrous magnesium sulfate. Then, the solvent was distilled off, and the obtained crude product was purified by preparative thin-layer silica gel column chromatography (ethyl acetate : hexane = 1 : 1) to obtain PAY15 (4.0 mg, 85%).

PAY15: ¹H-NMR δ: 8.76 (d, J = 1.6 Hz, 1H), 8.73 (d, J = 1.8 Hz, 1H), 8.17 (d, J = 1.0 Hz, 1H), 7.19 (d, J = 0.9 Hz, 2H), 3.91 (t, J = 5.8 Hz, 2H), 2.96 (t, J = 7.4 Hz, 2H), 2.86 (t, J = 6.2 Hz, 2H), 2.69 (t, J = 7.4 Hz, 2H), 1.98 (td, J = 6.2 Hz, 5.9 Hz, 2H)

### Example 3: Labeling and synthesis of [¹²⁵I]PAY15

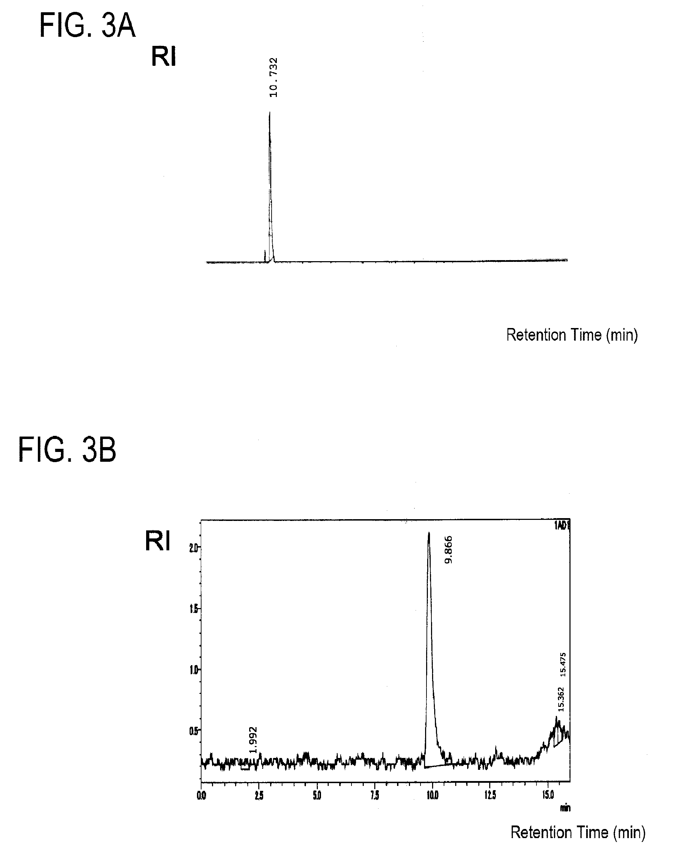

[¹²⁵I]PAY15 was synthesized via labeling according to the scheme shown in FIG. 2. Specifically, compound 3 (82 µg) obtained in Example 1 was treated with [¹²⁵I]sodium iodide (7.4 MBq) and N-chlorosuccinimide (11 µg) in methanol (100 µL) at room temperature (25°C) for 30 minutes and then separated and purified by high-performance liquid chromatography (hereinafter, abbreviated to "HPLC"). The obtained compound was confirmed to be [¹²⁵I]PAY15 on the basis of the complete agreement between its retention time and the HPLC retention time of the unlabeled form obtained in Example 2. This compound had a radiochemical yield of 50% relative to [¹²⁵I]sodium iodide, a radiochemical purity of 99% or higher, and a specific radioactivity of approximately 2.2 TBq/mmol. The analytical chart of the radiochemical purity using HPLC is shown in FIG. 3A.
[HPLC conditions]
Column: Cosmosil 5C₁₈-AR-II 10 x 250 mm
Mobile phase: acetonitrile (0.1 vol% trifluoroacetic acid) : water (0.1 vol% trifluoroacetic acid) = 70 : 30 (volume ratio)
RI detector: scintillation survey meter, Hitachi-Aloka Medical Ltd.
UV detector: wavelength of 254 nm, SPD-20A, Shimadzu Corp.
Flow rate: 2.0 mL/min
Retention time: 10.7 minutes

### Evaluation 1: Pharmacokinetic and distribution experiment

The HPLC fraction of [¹²⁵I]PAY15 obtained in Example 3 was concentrated and then diluted with saline. The resulting solution was used in administration. 100 µL of the solution (18.5 kBq (0.5 µCi)) was injected to the tail vein of each of five ddy mice (6 weeks old). Five minutes later, each mouse was decapitated, and blood was collected. Then, organs (adrenal gland, pancreas, heart, lung, stomach, small intestine, large intestine, liver, spleen, kidney, and thyroid gland) were excised. After measurement of their weights, the radioactivities of blood and each excised organ were measured. Also, the point in time when each mouse was decapitated was changed to 5, 15, 30, 60, and 120 minutes later to perform similar operation. Mean ± standard deviation of radioactivity distributions (%dose/g) in blood and each excised organ is shown in Table 1.

**Table 1**

| | | 5 min | 15 min | 30 min | 60 min | 120 min |
|---|---|---|---|---|---|---|
| Radioactivity Distribution (%dose/g) | Adrenal gland | 8.7±1.7 | 7.3±3.9 | 2.3±1.2 | 1.7±1.3 | 2.1±1.4 |
| | Pancreas | 4.0±0.9 | 2.8±0.1 | 1.7±0.1 | 1.5±0.1 | 1.3±0.3 |
| | Blood | 5.0±1.2 | 4.9±0.3 | 3.1±0.3 | 2.9±0.2 | 2.3±0.5 |
| | Heart | 3.6±0.8 | 2.6±0.5 | 1.3±0.1 | 1.0±0.0 | 0.8±0.2 |
| | Lung | 7.4±1.6 | 4.9±0.3 | 3.6±1.6 | 2.4±0.1 | 2.0±0.4 |
| | Stomach | 3.5±1.2 | 12.1±2.0 | 25.0±6.4 | 31.3±11.2 | 27.4±5.2 |
| | Small intestine | 3.7±0.9 | 4.6±0.9 | 4.0±0.4 | 4.0±0.4 | 3.0±0.6 |
| | Large intestine | 1.9±0.8 | 1.8±0.2 | 1.6±0.2 | 1.7±0.5 | 2.4±1.0 |
| | Liver | 5.1±1.1 | 3.0±0.3 | 1.5±0.2 | 1.2±0.1 | 1.0±0.2 |
| | Spleen | 4.1±0.8 | 3.2±0.3 | 2.2±0.3 | 1.6±0.2 | 1.4±0.2 |
| | Kidney | 6.1±1.2 | 4.5±0.4 | 3.5±0.9 | 2.7±0.6 | 2.7±1.6 |
| | Thyroid | 71.6±50.0 | 349. 4±115.9 | 486. 5±245.4 | 1298.8±729.1 | 1400.6±695.3 |
| | Adrenal gland/Blood ratio | 1.74±0.12 | 1.18±0.15 | 0.74±0.40 | 0.82±0.16 | 0.86±0.46 |
| | Adrenal gland/Liver ratio | 1.70±0.23 | 1.81±0.07 | 1.82±0.53 | 1.90±0.47 | 0.73±0.42 |
| | Adrenal gland/Kidney ratio | 1.42±0.03 | 1.24±0.17 | 0.75±0.20 | 0.84±0.21 | 0.87±0.42 |

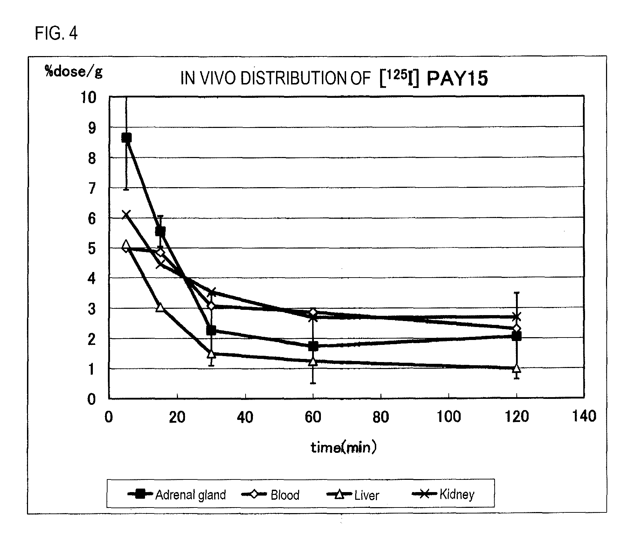

FIG. 4 shows time-dependent change in radioactivity accumulation in the adrenal gland, blood, liver, and kidney among the results shown in Table 1. At an early stage after administration, high radioactivity accumulation was observed in the adrenal gland compared with blood and periadrenal tissues.

### Example 4: Synthesis of labeling precursor of [¹²⁵I]PAY21

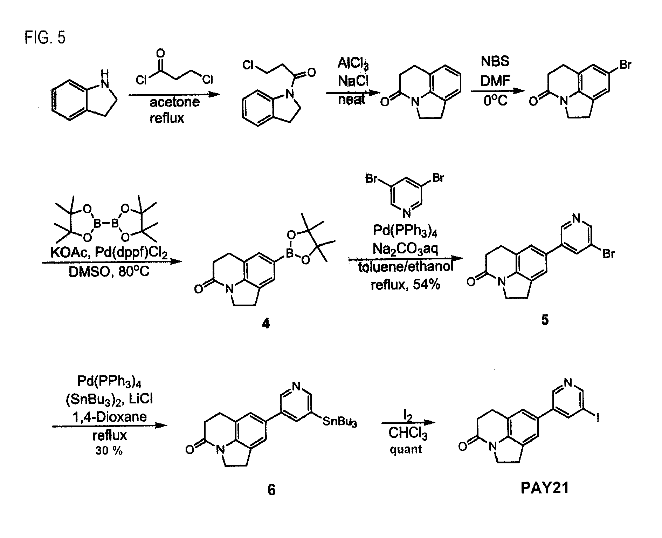

A labeling precursor of [¹²⁵I]PAY21 was synthesized according to the scheme shown in FIG. 5.

In FIGS. 2 and 5, the name of each compound is as follows:
Compound 4: 8-(boronic acid pinacol ester)-1,2,5,6-tetrahydro-4H-pyrrolo[3,2,1-ij]quinolin-4-one
Compound 5: 8-(5-bromopyridin-3-yl)-1,2,5,6-tetrahydro-4H-pyrrolo[3,2,1-ij]quinolin-4-one
Compound 6: 8-(5-tributylstannylpyridin-3-yl)-1,2,5,6-tetrahydro-4H-pyrrolo[3,2,1-ij]quinolin-4-one

### 1) Synthesis of compound 4

Compound 4 was synthesized according to a method described in Journal of Medicinal Chemistry, 2011, vol. 54, p. 2307-2319 with indoline as a starting material.

### 2) Synthesis of compound 5 from compound 4

In an argon atmosphere, a 1 mol/L aqueous sodium carbonate solution (860 µL, 8.6 mmol) and tetrakis(triphenylphosphine)palladium(0) (17 mg, 0.014 mmol) were added in this order to a toluene-ethanol (1.6/0.4 mL) mixed solution of compound 4 (43 mg, 0.14 mmol) and 3,5-dibromopyridine (51 mg, 0.21 mmol) under stirring at room temperature (25°C), and the mixture was stirred at the same temperature as above for 10 minutes and then stirred for 18 hours under heating to reflux. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (ethyl acetate : hexane (volume ratio) = 2 : 1) to obtain compound 5 (21 mg, yield relative to compound 4: 54%).

Compound 5: ¹H-NMR δ: 8.71 (d, J = 1.4 Hz, 1H), 8.60 (d, J = 1.6 Hz, 1H), 7.97 (d, J = 1.4 Hz, 1H), 7.29 (s, 2H), 3.91 (t, J = 5.8 Hz, 2H), 2.94 (t, J = 7.1 Hz, 2H), 2.84 (t, J = 5.8 Hz, 2H), 2.69 (t, J = 7.6 Hz, 2H)

### 3) Synthesis of compound 6 from compound 5

In an argon atmosphere, bistributyltin (55 µL, 0.11 mmol) and tetrakis(triphenylphosphine)palladium(0) (6.3 mg, 0.0055 mmol) were added in this order to a dioxane (2.0 mL) solution of compound 5 (18 mg, 0.055 mmol) and lithium chloride (6.9 mg, 0.16 mmol) under stirring at room temperature, and the mixture was stirred at the same temperature as above for 10 minutes and then stirred for 5 hours under heating to reflux. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer aminosilica gel column chromatography (ethyl acetate : hexane = 2 : 1) to obtain compound 6 (8.8 mg, 30%).

Compound 6: ¹H-NMR δ: 8.67 (d, J = 2.0 Hz, 1H), 8.54 (s, 1H), 7.84 (s, 1H), 7.20 (brs, 1H), 4.14 (t, J = 8.5 Hz, 2H), 3.25 (t, J = 8.5 Hz, 2H), 3.04 (t, J = 7.7 Hz, 2H), 2.73 (t, J = 7.7 Hz, 2H), 1.70-1.53 (m, 6H), 1.40-1.30 (m, 6H), 1.19-1.11 (m, 6H), 0.90 (t, J = 7.2 Hz, 9H)

### Example 5: Synthesis of PAY21 (unlabeled form)

An unlabeled form of PAY21 was synthesized according to the scheme shown in FIG. 5.

In a nitrogen atmosphere, iodine (1.4 mg, 0.011 mmol) was added to a chloroform (1.0 mL) solution of compound 6 (5.3 mg, 0.010 mmol) obtained in Example 4 under stirring at room temperature, and the mixture was stirred at the same temperature as above for 90 minutes. Then, a saturated aqueous solution of sodium thiosulfate was added thereto, followed by extraction three times with chloroform. The extract was washed with saturated saline and dried over anhydrous magnesium sulfate. Then, the solvent was distilled off, and the obtained crude product was purified by preparative thin-layer silica gel column chromatography (ethyl acetate : hexane = 2 : 1) to obtain PAY21 (3.6 mg, quant).

PAY21: ¹H-NMR δ: 8.76 (d, J = 1.6 Hz, 1H), 8.71 (d, J = 1.8 Hz, 1H), 8.15 (d, J = 1.0 Hz, 1H), 7.18 (d, J = 0.7 Hz, 1H), 4.14 (t, J = 8.5 Hz, 2H), 3.25 (t, J = 8.5 Hz, 2H), 3.04 (t, J = 7.8 Hz, 2H), 2.73 (t, J = 7.8 Hz, 2H)

### Example 6: Labeling and synthesis of [¹²⁵I]PAY21

[¹²⁵I]PAY15 was synthesized via labeling according to the scheme shown in FIG. 2. Specifically, compound 6 (82 µg) obtained in Example 4 was treated with [¹²⁵I]sodium iodide (7.4 MBq) and N-chlorosuccinimide (11 µg) in methanol (100 µL) at room temperature (25°C) for 30 minutes and then separated and purified by HPLC. The obtained compound was confirmed to be [¹²⁵I]PAY21 on the basis of the complete agreement between its retention time and the HPLC retention time of the unlabeled form obtained in Example 5. This compound had a radiochemical yield of 50% relative to [¹²⁵I]sodium iodide, a radiochemical purity of 99% or higher, and a specific radioactivity of approximately 2.2 TBq/mmol. The analytical chart of the radiochemical purity using HPLC is shown in FIG. 3B.
[HPLC conditions]
Column: Cosmosil 5C₁₈-AR-II 10 x 250 mm
Mobile phase: acetonitrile (0.1 vol% trifluoroacetic acid) : water (0.1 vol% trifluoroacetic acid) = 60 : 40
(volume ratio)
RI detector: scintillation survey meter, Hitachi-Aloka Medical Ltd.
UV detector: wavelength of 254 nm, SPD-20A, Shimadzu Corp.
Flow rate: 2.0 mL/min
Retention time: 9.9 minutes

### Evaluation 2-1: Pharmacokinetic and distribution experiment

The HPLC fraction of [¹²⁵I]PAY21 obtained in Example 6 was concentrated and then diluted with saline. The resulting solution was used in administration to perform the same operation as in Evaluation 1. Mean ± standard deviation of radioactivity distributions (%dose/g) in blood and each excised organ is shown in Table 2.

**Table 2**

| | | 5 min | 15 min | 30min | 60min | 120 min |
|---|---|---|---|---|---|---|
| Redioactivily Distribution (%dose/g) | Adrenal gland | 9.7±1.4 | 3.6±1.0 | 2.8±0.3 | 1.9±0.8 | 1.8±0.7 |
| | Pancreas | 4.0±0.5 | 2.6±0.3 | 2.4±0.5 | 1.7±0.3 | 1.6±0.1 |
| | Blood | 5.7±0.8 | 4.7±0.4 | 3.7±0.4 | 3.4±0.6 | 2.6±0.3 |
| | Heart | 3.6±0.4 | 2.1±0.3 | 1.5±0.1 | 1.3±0.2 | 1.0±0.2 |
| | Lung | 5.6±0.7 | 4.1±0.3 | 3.4±03. | 3.1±0.6 | 2.6±0.7 |
| | Stomach | 10.1±1.6 | 28.4±3.3 | 46.7±3.5 | 39.4±9.1 | 38.6±6.5 |
| | Small intestine | 6.0±1.0 | 8.1±2.2 | 10.3±2.8 | 10.9±2.9 | 4.8±1.3 |
| | Large intestine | 2.3±0.4 | 2.2±0.1 | 2.1±0.2 | 2.0±0.4 | 10.0±4.8 |
| | Liver | 8.6±1.1 | 3.2±0.4 | 2.2±0.2 | 1.6±0.3 | 1.4±0.2 |
| | Spleen | 3.9±0.4 | 3.0±0.4 | 2.5±0.6 | 1.9±0.4 | 1.7±0.1 |
| | Kidney | 6.4±0.4 | 4.0±0.6 | 3.4±0.4 | 2.9±0.6 | 2.4±0.6 |
| | Thyroid | 114.1±22.4 | 332.2±43.1 | 1570.0±165.0 | 1318.7±800.8 | 4664.0±1419.1 |
| | Adrenal gland/Blood ratio | 1.71±0.08 | 0.75±0.17 | 0.76±0.14 | 0.54±0.17 | 0.71±0.32 |
| | Adrenal gland/Liver ratio | 1.13±0.14 | 1.12±0.36 | 1.26±0.20 | 1.15±0.38 | 1.33±0.64 |
| | Adrenal gland/Kidney ratio | 1.52±0.19 | 0.90±0.24 | 0.82±0.10 | 0.65±0.21 | 0.81±0.44 |

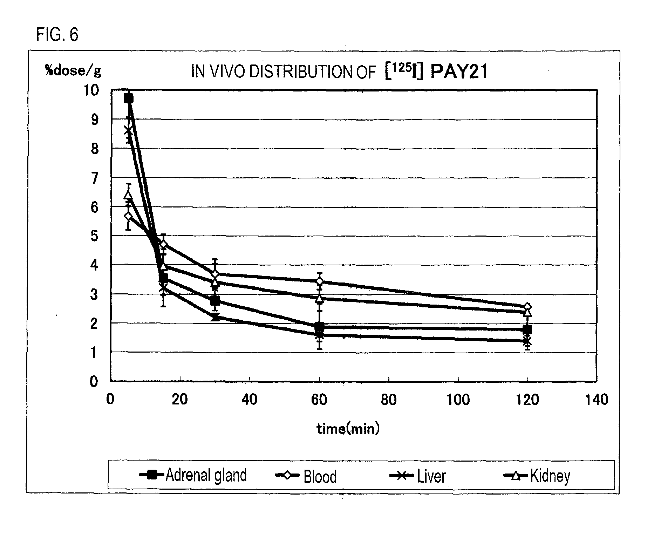

FIG. 6 shows time-dependent changes in radioactivity accumulation in the adrenal gland, blood, liver, and kidney among the results shown in Table 2. At an early stage after administration, high radioactivity accumulation was observed in the adrenal gland compared with blood and periadrenal tissues, as in the case of [¹²⁵I]PAY15. Evaluation was conducted by similar operation using CD-1 (ICR) mice instead of the ddy mice, showing no difference between the species.

### Evaluation 2-2: Stability evaluation

The stability of [¹²⁵I]PAY21 in plasma was evaluated by the following procedures: Blood was collected from the heart of each CD-1 mouse. Plasma was separated by centrifugation and passed through Cosmonice filter (diameter: 4 mm, pore size: 0.45 µm, Nacalai Tesque, Inc.). 350 µL of the plasma thus filtered was incubated at 37°C for 15 minutes. Then, 30 µL of a saline solution of [¹²⁵I]PAY21 (18.5 kBq (0.5 µCi)) was added thereto. After incubation for 120 minutes, a 100 µL aliquot of the mixture was separated and added to methanol (500 µL) under ice cooling. The mixture was further centrifuged, and the supernatant (400 µL) was analyzed by HPLC. The HPLC conditions were the same as those shown in Example 6.

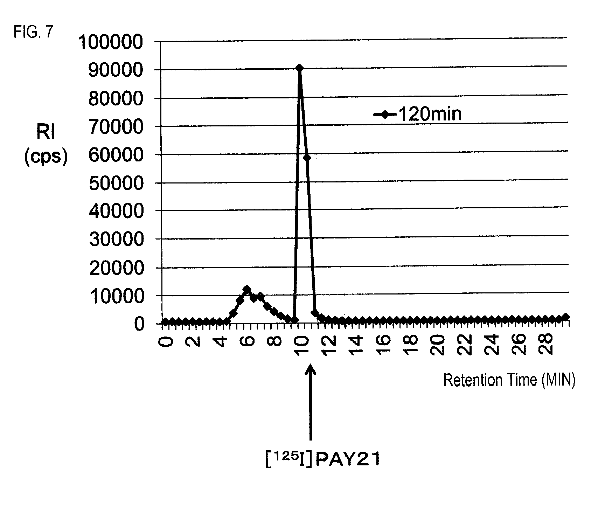

The results obtained using an RI detector are shown in FIG. 7. [¹²⁵I]PAY21 (retention time: 9.9 minutes) had a peak area of approximately 60%, demonstrating that [¹²⁵I]PAY21 is generally stable in plasma.

### Example 7: Synthesis of labeling precursor of [¹⁸F]PAY27

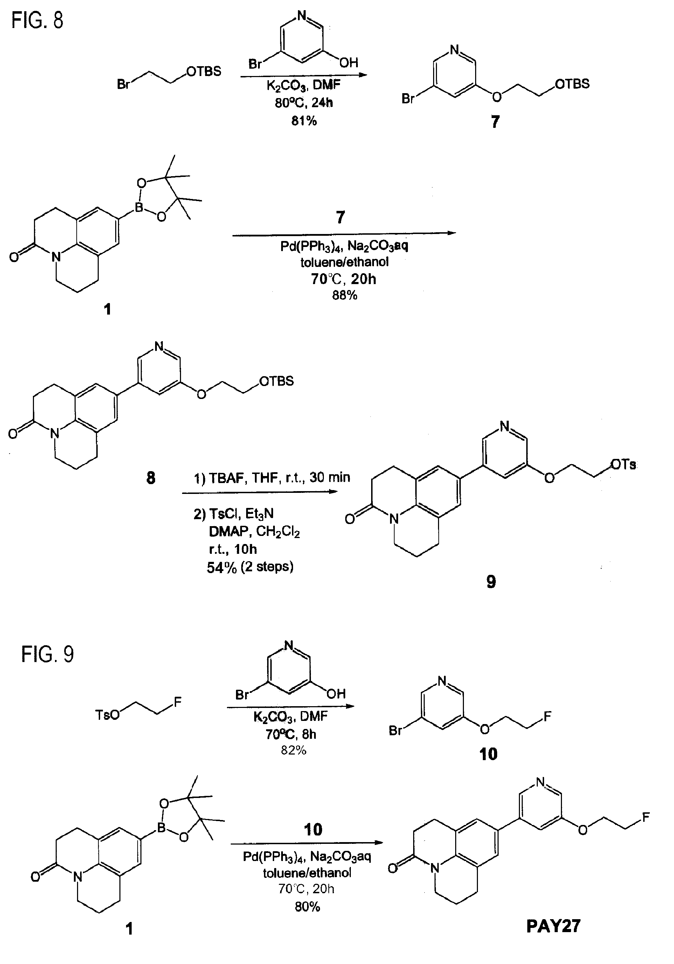

A labeling precursor of [¹⁸F]PAY27 was synthesized according to the scheme shown in FIG. 8.

In FIG. 8, the name of each compound is as follows: Compound 1: the same as in Example 1
Compound 7: 3-bromo-5-(2-tert-butyldimethylsilyloxyethyloxy)pyridine
Compound 8: 9-(5-(2-tert-butyldimethylsilyloxyethyloxy)pyridin-3-yl)-1,2,6,7-tetrahydro-5H-pyrido[3,2,1-ij]quinolin-3-one
Compound 9: 9-(5-(2-(4-methylbenzenesulfonyl)oxy)ethyloxy)pyridin-3-yl)-1,2,6,7-tetrahydro-5H-pyrido[3,2,1-ij]quinolin-3-one

### 1) Synthesis of compound 1

The compound was synthesized in the same way as in Example 1.

### 2) Synthesis of compound 7

In an argon atmosphere, potassium carbonate (1.1 g, 8.2 mmol) was added to a N,N-dimethylformaldehyde (15 mL) solution of 3-bromo-5-hydroxypyridine (710 mg, 4.1 mmol) and (2-bromoethoxy)-tert-butyldimethylsilane (1.0 mL, 4.9 mmol) under stirring at room temperature (25°C), and the mixture was stirred at the same temperature as above for 10 minutes and then stirred at 80°C for 24 hours. After the completion of the reaction, distilled water was added to the reaction mixture, followed by extraction four times with diethyl ether. The extract was washed with saturated saline and dried over anhydrous magnesium sulfate. Then, the solvent was distilled off, and the obtained crude product was purified by medium-pressure flash column chromatography (ethyl acetate : hexane (volume ratio) = 1 : 99 → 1 : 4) to obtain compound 7 (1.1 g, yield relative to 3-bromo-5-hydroxypyridine: 81%) in a brown solid form.

Compound 7: ¹H-NMR δ: 8.27 (d, J = 1.6 Hz, 1H), 8.25 (d, J = 1.6 Hz, 1H), 7.41 (t, J = 1.9 Hz, 1H), 4.09 (t, J = 4.8 Hz, 2H), 3.98 (t, J = 4.8 Hz, 2H), 0.90 (s, 9H), 0.09 (s, 6H)

### 3) Synthesis of compound 8 from compounds 1 and 7

In an argon atmosphere, a 1 mol/L aqueous sodium carbonate solution (957 µL, 0.96 mmol) and tetrakis(triphenylphosphine)palladium(0) (18 mg, 0.016 mmol) were added in this order to a toluene-ethanol (2.0/0.5 mL) mixed solution of compound 1 (50 mg, 0.16 mmol) and compound 7 (64 µL, 0.24 mmol) under stirring at room temperature (25°C), and the mixture was stirred at the same temperature as above for 10 minutes and then stirred at 70°C for 20 hours. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (ethyl acetate : hexane (volume ratio) = 2 : 1) to obtain compound 8 (60 mg, yield relative to compound 1: 88%) in a colorless solid form.

Compound 8: ¹H-NMR δ: 8.32 (d, J = 1.8 Hz, 1H), 8.18 (d, J = 2.7 Hz, 1H), 7.37 (d, J = 2.7 Hz, 1H), 7.12 (d, J = 1.8 Hz, 2H), 4.07 (t, J = 5.0 Hz, 2H), 3.92 (t, J = 5.0 Hz, 2H), 3.82 (t, J = 6.0 Hz, 2H), 2.87 (t, J = 7.5 Hz, 2H), 2.77 (t, J = 6.2 Hz, 2H), 2.61 (t, J = 7.5 Hz, 2H), 1.90 (td, J = 6.2 Hz, 6.0 Hz, 2H), 0.82 (s, 9H), 0.02 (s, 6H)

### 4) Synthesis of compound 9 from compound 8

In an argon atmosphere, a 1.0 mol/L solution of tetrabutylammonium fluoride in tetrahydrofuran (85 µL, 0.85 mmol) was added to a tetrahydrofuran (1.0 mL) solution of compound 8 (30 mg, 0.07 mmol) under stirring at 0°C, and the mixture was stirred at room temperature (25°C) for 30 minutes. After the completion of the reaction, distilled water was added to the reaction solution, followed by extraction with ethyl acetate. The extract was washed with saturated saline and dried over anhydrous magnesium sulfate. Then, the solvent was distilled off to obtain a colorless oil (20 mg).

In an argon atmosphere, p-toluenesulfonic acid chloride (15 mg, 0.076 mmol) and N,N-dimethylaminopyridine (6 mg, 0.006 mmol) were added to a methylene chloride (2.0 mL) suspension of the obtained colorless oil (20 mg) and triethylamine (18 µL, 0.13 mmol) under stirring at 0°C, and the mixture was stirred at room temperature (25°C) for 10 hours. After the completion of the reaction, the reaction solution was poured into a saturated aqueous solution of sodium bicarbonate, followed by extraction with ethyl acetate. The extract was washed with saturated saline and dried over anhydrous magnesium sulfate. Then, the solvent was distilled off, and the obtained crude product was purified by preparative thin-layer silica gel column chromatography (chloroform : methanol (volume ratio) = 20 : 1) to obtain compound 9 (16 mg, yield relative to compound 8: 54%).

Compound 9 :¹H-NMR δ: 8.44 (s, 1H), 8.14 (d, J = 2.5 Hz, 1H), 7.82 (d, J = 8.2 Hz, 2H), 7.36 (s, 1H), 7.34 (s, 2H), 7.20 (s, 2H), 4.42 (dd, J = 1.8 Hz, 3.0 Hz, 2H), 4.29 (dd, J = 1.8 Hz, 3.0 Hz, 2H), 3.91 (t, J = 6.0 Hz, 2H), 2.95 (t, J = 7.5 Hz, 2H), 2.86 (t, J = 6.2 Hz, 2H), 2.44 (s, 3H), 1.97 (td, J = 6.2 Hz, 6.0 Hz, 2H)

### Example 8: Synthesis of PAY27 (unlabeled form)

An unlabeled form of [¹⁸F]PAY27 was synthesized according to the scheme shown in FIG. 9.

In FIG. 9, the name of each compound is as follows:
Compound 1: the same as in Example 1
Compound 10: 2-bromo-5-(2-fluoroethyloxy)pyridine

### 1) Synthesis of compound 1

The compound was synthesized in the same way as in Example 1.

### 2) Synthesis of compound 10

In an argon atmosphere, potassium carbonate (1.6 g, 11.3 mmol) was added to a N,N-dimethylformamide (15 mL) solution of 3-bromo-5-hydroxypyridine (980 mg, 5.6 mmol) and 2-fluoroethyl-4-methylbenzenesulfonate (1.2 ml, 6.8 mmol) under stirring at room temperature (25°C), and the mixture was stirred at the same temperature as above for 10 minutes and then stirred at 70°C for 8 hours. After the completion of the reaction, distilled water was added to the reaction mixture, followed by extraction four times with diethyl ether. The extract was washed with saturated saline and dried over anhydrous magnesium sulfate. Then, the solvent was distilled off, and the obtained crude product was purified by silica gel column chromatography (ethyl acetate : hexane (volume ratio) = 1 : 5) to obtain compound 10 (1.0 g, yield relative to 3-bromo-5-hydroxypyridine: 82%) in a pale yellow liquid form.

Compound 10: ¹H-NMR δ: 8.28 (d, 1H), 8.22 (d, 1H), 7.30 (t, 1H), 4.75 (t, 1H), 4.60 (t, 1H), 4.28 (t, 1H), 4.17 (t, 1H)

### 3) Synthesis of PAY27 from compounds 1 and 10

In an argon atmosphere, a 1 mol/L aqueous sodium carbonate solution (957 µL, 0.96 mmol) and tetrakis(triphenylphosphine)palladium(0) (18 mg, 0.016 mmol) were added in this order to a toluene-ethanol (2.0/0.5 mL) mixed solution of compound 1 (50 mg, 0.16 mmol) and compound 10 (35 µg, 0.24 mmol) under stirring at room temperature (25°C), and the mixture was stirred at the same temperature as above for 10 minutes and then stirred at 70°C for 20 hours. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (ethyl acetate : hexane (volume ratio) = 2 : 1) to obtain PAY27 (40 mg, yield relative to compound 1: 80%) in a colorless solid form.

PAY27: ¹H-NMR δ: 8.46 (d, J = 1.4 Hz, 1H), 8.39 (d, J = 2.7 Hz, 1H), 7.38 (d, J = 2.7 Hz, 1H), 7.22 (d, J = 1.4 Hz, 2H), 4.80 (dt, J_{F} = 47.4 Hz, J = 4.0 Hz, 2H), 4.34 (dt, J_{F} = 27.6 Hz, J = 4.0 Hz, 2H), 3.91 (t, J = 5.8 Hz, 2H), 2.96 (dd, J = 7.8 Hz, 7.1 Hz, 2H), 2.87 (t, J = 6.2 Hz, 2H), 2.70 (dd, J = 7.8 Hz, 7.1 Hz, 2H), 1.99 (td, J = 6.2 Hz, 6.0 Hz, 2H)

### Example 9: Study on labeling and synthesis of [¹⁸F]PAY27

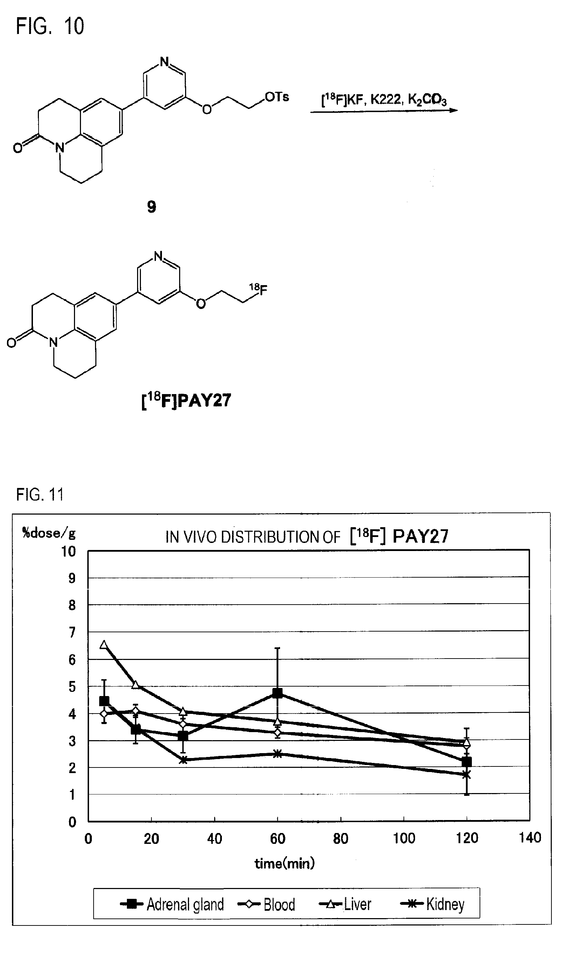

[¹⁸F]PAY27 was synthesized via labeling according to the scheme shown in FIG. 10. Specifically, an acetonitrile (300 µL) solution of Kryptofix 222 (trade name, 2.0 mg) was added to a potassium carbonate-water solution of [¹⁸F]potassium fluoride (1 µL, 3.7 to 7.4 MBq (1 to 2 mCi), potassium carbonate concentration: 66 mmol/mL), followed by azeotropic dehydration three times at 120°C in an argon atmosphere. To the residue, an acetonitrile or dimethyl sulfoxide (200 µL) solution of compound 9 (1.0 mg) obtained in Example 7 was added, and the mixture was heated at a temperature for a time as shown in Table 3. After cooling to room temperature (25°C), each reaction solution was applied to HPLC to separate and purify a compound. The obtained compound was confirmed to be [¹⁸F]PAY27 on the basis of the complete agreement between its retention time and the HPLC retention time of the unlabeled form obtained in Example 8. This compound had a radiochemical yield as shown in Table 3 relative to [¹⁸F]potassium fluoride and a radiochemical purity of 99% or higher as a result of confirmation by HPLC.
[Preparative HPLC conditions]
Column: Cosmosil 5C₁₈-AR-II, 10 x 250 mm
Mobile phase: acetonitrile : 20 mmol/L phosphate buffer solution (pH 2.5) = 35 : 65 (volume ratio)
RI detector: US-3000 radioHPLC detector, Universal Giken Co., Ltd.
UV detector: wavelength of 254 nm, SPD-20A, Shimadzu Corp.
Flow rate: 4.0 mL/min
Retention time: 9.0 minutes
[Analytical HPLC conditions]
Column: Cosmosil 5C₁₈-AR-II, 4.6 x 150 mm
Mobile phase: acetonitrile : 20 mmol/L phosphate buffer solution (pH 2.5) = 50 : 50 (volume ratio)
RI detector: US-3000 radioHPLC detector, Universal Giken Co., Ltd.
UV detector: wavelength of 254 nm, SPD-20A, Shimadzu Corp.
Flow rate: 1.0 mL/min
Retention time: 3.0 minutes

**Table 3**

| No. | Solvent | Temperature | Reaction time | Radiochemical yield |
|---|---|---|---|---|
| **1** | Acetonitrile | **100°C** | 10min | **3%** |
| **2** | Acetonitrile | **120°C** | 10min | **6%** |
| **3** | Acetonitrile | **120°C** | 20min | **9%** |
| **4** | Acetonitrile | **140°C** | 10min | **11%** |
| **5** | Dimethyl Sulfoxide | **130°C** | 10min | **36%** |
| **6** | Dimethyl Sulfoxide | **140°C** | 10min | **86%** |
| **7** | Dimethyl Sulfoxide | **150°C** | 10min | **73%** |
| **8** | Dimethyl Sulfoxide | **160°C** | 10min | **58%** |

### Example 10: Labeling and synthesis of [¹⁸F]PAY27

[¹⁸F]PAY27 was synthesized via labeling according to the method of Experimental No. 6 of Example 9 except that a potassium carbonate-acetonitrile-water mixed solution of [¹⁸F]potassium fluoride (10 µL, 37 MBq (10 mCi)) was used. A compound was separated and purified by high-performance liquid chromatography (HPLC). The obtained compound was confirmed to be [¹⁸F]PAY27 on the basis of the complete agreement between its retention time and the HPLC retention time of the unlabeled form obtained in Example 8. This compound had a radiochemical yield of 86% relative to [¹⁸F]potassium fluoride and a radiochemical purity of 99% or higher. The fraction from HPLC was diluted with purified water (30 mL) and then passed through Sep-Pak Plus PS-2 cartridge (pretreated with acetonitrile (5 mL) and purified water (5 mL)). The cartridge was further washed with purified water (10 mL). The cartridge was dried in argon gas, followed by elution with acetonitrile (3 mL). The eluate was concentrated under reduced pressure at 40°C.

### Evaluation 3: Pharmacokinetic and distribution experiments

[¹⁸F]PAY27 obtained in Example 10 was diluted with saline. The resulting solution was used in administration. 100 µL of the solution (14.8 kBq (4 µCi)) was injected to the tail vein of each of five ddy mice (6 weeks old). Five minutes later, each mouse was decapitated, and blood was collected. Then, organs (adrenal gland, pancreas, heart, lung, stomach, small intestine, large intestine, liver, spleen, kidney, and thigh bone) were excised. After measurement of their weights, the radioactivities of blood and each excised organ were measured. Also, the point in time when each mouse was decapitated was changed to 5, 15, 30, 60, and 120 minutes later to perform similar operation. Mean ± standard deviation of radioactivity distributions (%dose/g) in blood and each excised organ is shown in Table 4.

**Table 4**

| | | 5 min | 15 min | 30 min | 60 min | 120 min |
|---|---|---|---|---|---|---|
| Radioactivity Distribution (%dose/g) | Adrenal gland | 4.5±0.8 | 3.4±0.5 | 3.2±0.6 | 4.7±1.7 | 2.2±1.2 |
| | Pancreas | 3.0±0.4 | 2.5±0.2 | 2.0±0.1 | 2.0±0.1 | 1.5±0.2 |
| | Blood | 4.0±0.3 | 4.1±0.2 | 3.6±0.3 | 3.3±0.1 | 2.8±0.5 |
| | Heart | 3.9±0.3 | 3.6±0.2 | 3.2±0.4 | 3.0±0.2 | 2.4±0.3 |
| | Lung | 3.7±0.4 | 3.6±0.1 | 2.7±0.2 | 2.6±0.2 | 2.1±0.1 |
| | Stomach | 5.1±0.4 | 5.6±1.1 | 5.5±0.4 | 4.1±0.9 | 3.9±0.6 |
| | Small intestine | 5.0±0.4 | 6.7±0.6 | 5.9±0.5 | 6.0±0.7 | 3.1±0.6 |
| | Large intestine | 2.5±0.2 | 5.0±0.4 | 6.1±0.4 | 7.0±0.6 | 9.1±1.4 |
| | Liver | 6.5±1.0 | 5.1±0.3 | 4.1±0.5 | 3.7±0.3 | 2.9±0.5 |
| | Spleen | 3.0±0.2 | 2.8±0.2 | 2.3±0.1 | 2.2±0.2 | 1.8±0.2 |
| | Kidney | 4.5±0.3 | 3.5±0.3 | 2.3±0.2 | 2.5±0.4 | 1.7±0.1 |
| | Femur | 1.8±0.1 | 2.3±0.1 | 2.4±0.2 | 3.9±0.2 | 6.4±0.4 |
| | Adrenal gland/Blood ratio | 1.11±0.17 | 0.83±0.12 | 0.88±0.18 | 1.43±0.47 | 0.58±0.44 |
| | Adrenal gland/Liver ratio | 0.70±0.16 | 0.67±0.06 | 0.79±0.20 | 1.26±0.37 | 0.58±0.46 |
| | Adrenal gland/Kidney ratio | 1.00±0.19 | 0.99±0.23 | 1.40±0.28 | 1.88±0.55 | 0.99±0.77 |

FIG. 11 shows time-dependent changes in radioactivity accumulation in the adrenal gland, blood, liver, and kidney among the results shown in Table 4. At 60 minutes after administration, high radioactivity accumulation was observed in the adrenal gland compared with blood and periadrenal tissues.

### Example 11: Synthesis of labeling precursor of [¹⁸F]PAY54

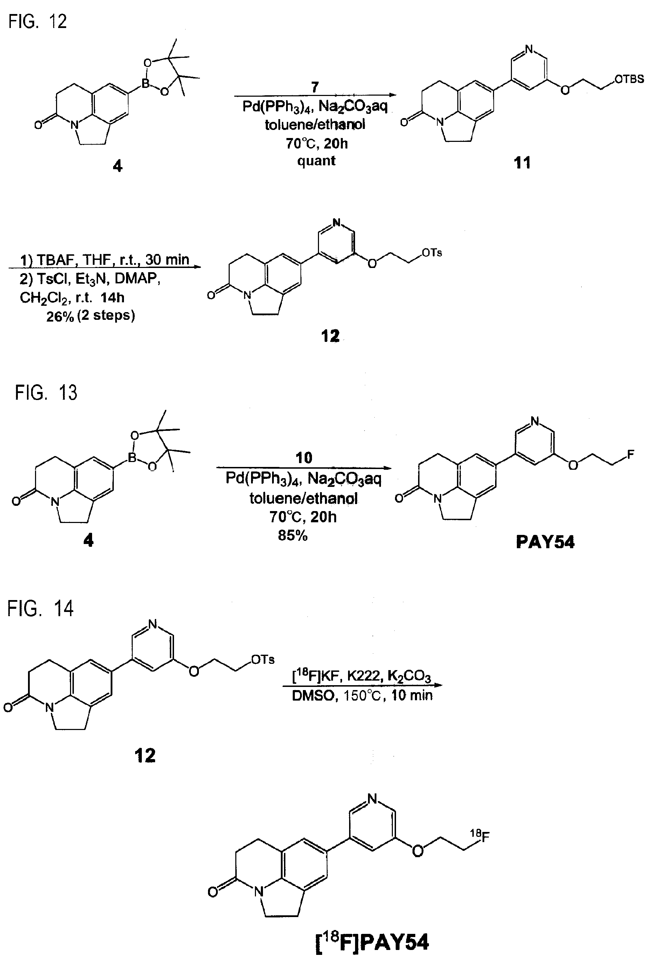

A labeling precursor of [¹⁸F]PAY54 was synthesized according to the scheme shown in FIG. 12.

In FIG. 12, the name of each compound is as follows:
Compound 4: the same as in Example 4
Compound 7: the same as in Example 7
Compound 11: 8-(5-(2-tert-butyldimethylsilyloxyethyloxy)pyridin-3-yl)-1,2,5,6-tetrahydro-4H-pyrrolo[3,2,1-ij]quinolin-4-one Compound 12: 8-(5-(2-(4-methylbenzenesulfonyl)oxy)ethyloxy)pyridin-3-yl)-1,2,5,6-tetrahydro-4H-pyrrolo[3,2,1-ij]quinolin-4-one

### 1) Synthesis of compound 4

The compound was synthesized in the same way as in Example 4.

### 2) Synthesis of compound 7

The compound was synthesized in the same way as in Example 7.

### 3) Synthesis of compound 11 from compounds 4 and 7

In an argon atmosphere, a 1 mol/L aqueous sodium carbonate solution (1.0 mL, 1.0 mmol) and tetrakis(triphenylphosphine)palladium(0) (19 mg, 0.017 mmol) were added in this order to a toluene-ethanol (2.0/0.5 mL) mixed solution of compound 4 (50 mg, 0.17 mmol) and compound 7 (19 mg, 0.25 mmol) under stirring at room temperature (25°C), and the mixture was stirred at the same temperature as above for 10 minutes and then stirred at 70°C for 20 hours. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (chloroform : methanol (volume ratio) = 20 : 1) to obtain compound 11 (70 mg, yield relative to compound 4: quant).

Compound 11: ¹H-NMR δ: 8.32 (d, J = 1.8 Hz, 1H), 8.18 (d, J = 2.7 Hz, 1H), 7.37 (d, J = 2.7 Hz, 1H), 7.12 (d, J = 1.8 Hz, 2H), 4.14 (t, J = 5.0 Hz, 2H), 3.92 (t, J = 5.0 Hz, 2H), 3.82 (t, J = 6.0 Hz, 2H), 3.25 (t, J = 7.5 Hz, 2H), 2.77 (t, J = 6.2 Hz, 2H), 2.61 (t, J = 7.5 Hz, 2H), 0.82 (s, 9H), 0.02 (s, 6H)

### 4) Synthesis of compound 12 from compound 11

In an argon atmosphere, a 1.0 mol/L solution of tetrabutylammonium fluoride in tetrahydrofuran (300 µL, 0.30 mmol) was added to a tetrahydrofuran (2.0 mL) solution of compound 11 (70 mg, 0.17 mmol) under stirring at 0°C, and the mixture was stirred at room temperature (25°C) for 30 minutes. After the completion of the reaction, distilled water was added to the reaction solution, followed by extraction with ethyl acetate. The extract was washed with saturated saline and dried over anhydrous magnesium sulfate. Then, the solvent was distilled off to obtain an alcohol form (53 mg).

In an argon atmosphere, p-toluenesulfonic acid chloride (34 mg, 0.18 mmol) and N,N-dimethylaminopyridine (1.8 mg, 0.015 mmol) were added to a methylene chloride (2.0 mL) suspension of the obtained alcohol form (53 mg) and triethylamine (42 µL, 0.30 mmol) under stirring at 0°C, and the mixture was stirred at room temperature (25°C) for 14 hours. After the completion of the reaction, the reaction solution was poured into a saturated aqueous solution of sodium bicarbonate, followed by extraction with ethyl acetate. The extract was washed with saturated saline and dried over anhydrous magnesium sulfate. Then, the solvent was distilled off, and the obtained crude product was purified by preparative thin-layer silica gel column chromatography (chloroform : methanol (volume ratio) = 20 : 1) to obtain compound 12 (20 mg, yield relative to compound 11: 26%).

Compound 12: ¹H-NMR δ: δ: 8.44 (s, 1H), 8.14 (d, J = 2.5 Hz, 1H), 7.82 (d, J = 8.2 Hz, 2H), 7.36 (s, 1H), 7.34 (s, 2H), 7.20 (s, 2H), 4.42 (dd, J = 1.8 Hz, 3.0 Hz, 2H), 4.29 (dd, J = 1.8 Hz, 3.0 Hz, 2H), 4.14 (t, J = 6.0 Hz, 2H), 3.25 (t, J = 7.5 Hz, 2H), 2.86 (t, J = 6.2 Hz, 2H), 2.44 (s, 3H)

### Example 12: Synthesis of PAY54 (unlabeled form)

An unlabeled form of [¹⁸F]PAY54 was synthesized according to the scheme shown in FIG. 13.

In FIG. 13, the name of each compound is as follows: Compound 4: the same as in Example 4

### Compound 10: the same as in Example 8

### 1) Synthesis of compound 4

The compound was synthesized in the same way as in Example 4.

### 2) Synthesis of compound 10

The compound was synthesized in the same way as in Example 8.

### 3) Synthesis of PAY54 from compounds 4 and 10

In an argon atmosphere, a 1 mol/L aqueous sodium carbonate solution (600 µL, 0.60 mmol) and tetrakis(triphenylphosphine)palladium(0) (12 mg, 0.010 mmol) were added in this order to a toluene-ethanol (1.6/0.4 mL) mixed solution of compound 4 (30 mg, 0.10 mmol) and compound 10 (22 µg, 0.15 mmol) under stirring at room temperature (25°C), and the mixture was stirred at the same temperature as above for 10 minutes and then stirred at 70°C for 20 hours. After the completion of the reaction, distilled water was added to the reaction mixture, followed by extraction three times with ethyl acetate. The extract was washed with saturated saline and dried over anhydrous magnesium sulfate. Then, the solvent was distilled off, and the obtained crude product was purified by preparative thin-layer silica gel column chromatography (chloroform : methanol (volume ratio) = 30 : 1) to obtain PAY54 (27 mg, yield relative to compound 4: 85%).

PAY54: ¹H-NMR δ: 8.46 (d, J = 1.4 Hz, 1H), 8.39 (d, J = 2.7 Hz, 1H), 7.38 (d, J = 2.7 Hz, 1H), 7.22 (d, J = 1.4 Hz, 2H), 4.80 (dt, J_{F} = 47.4 Hz, J = 4.0 Hz, 2H), 4.34 (dt, J_{F} = 27.6 Hz, J = 4.0 Hz, 2H), 4.14 (t, J = 5.8 Hz, 2H), 3.25 (dd, J = 7.8 Hz, 7.1 Hz, 2H), 2.87 (t, J = 6.2 Hz, 2H), 2.70 (dd, J = 7.8 Hz, 7.1 Hz, 2H)

### Example 13: Labeling and synthesis of [¹⁸F]PAY54

[¹⁸F]PAY54 was synthesized via labeling according to the scheme shown in FIG. 14. Specifically, an acetonitrile (300 µL) solution of Kryptofix 222 (trade name, 2.0 mg) was added to a potassium carbonate-water solution of [¹⁸F]potassium fluoride (1 µL, 3.7 MBq (1 mCi), potassium carbonate concentration: 66 mmol/mL), followed by azeotropic dehydration three times at 120°C in an argon atmosphere. To the residue, a dimethyl sulfoxide (200 µL) solution of compound 12 (1.0 mg) was added, and the mixture was heated at 150°C for 10 minutes. After cooling to room temperature (25°C), the reaction solution was applied to HPLC to separate and purify a compound. The obtained compound was confirmed to be [¹⁸F]PAY54 on the basis of the complete agreement between its retention time and the HPLC retention time of the unlabeled form obtained in Example 12. This compound had a radiochemical yield of 61% relative to [¹⁸F]potassium fluoride and a radiochemical purity of 99% as a result of confirmation by HPLC.
[Preparative HPLC conditions]
Column: Cosmosil 5C₁₈-AR-II, 10 x 250 mm
Mobile phase: acetonitrile : 20 mmol/L phosphate buffer solution (pH 2.5) = 35 : 65 (volume ratio)
RI detector: US-3000 radioHPLC detector, Universal Giken Co., Ltd.
UV detector: wavelength of 254 nm, SPD-20A, Shimadzu Corp.
Flow rate: 4.0 mL/min
Retention time: 5.0 minutes
[Analytical HPLC conditions]
Column: Cosmosil 5C₁₈-AR-II, 4.6 x 150 mm
Mobile phase: acetonitrile : 20 mmol/L phosphate buffer solution (pH 2.5) = 35 : 65 (volume ratio)
RI detector: US-3000 radioHPLC detector, Universal Giken Co., Ltd.
UV detector: wavelength of 254 nm, SPD-20A, Shimadzu Corp.
Flow rate: 1.0 mL/min
Retention time: 4.0 minutes

A fraction from HPLC was diluted with purified water (30 mL) and then passed through Sep-Pak Plus PS-2 cartridge (pretreated with acetonitrile (5 mL) and purified water (5 mL)). The cartridge was further washed with purified water (10 mL). The cartridge was dried in argon gas, followed by elution with acetonitrile (3 mL). The eluate was concentrated under reduced pressure at 40°C.

### Evaluation 4: Pharmacokinetic and distribution experiments

[¹⁸F]PAY54 obtained in Example 13 was diluted with saline. The resulting solution was used in administration. 100 µL of the solution (14.8 kBq (4 µCi)) was injected to the tail vein of each of five ddy mice (6 weeks old). Five minutes later, each mouse was decapitated, and blood was collected. Then, organs (adrenal gland, pancreas, heart, lung, stomach, small intestine, large intestine, liver, spleen, kidney, and thigh bone) were excised. After measurement of their weights, the radioactivities of blood and each excised organ were measured. Also, the point in time when each mouse was decapitated was changed to 5, 15, 30, 60, and 120 minutes later to perform similar operation. Mean ± standard deviation of radioactivity distributions (%dose/g) in blood and each excised organ is shown in Table 5.

**Table 5**

| | | 5 min | 15 min | 30min | 60min | 120min |
|---|---|---|---|---|---|---|
| Radioactivity Distribution (%dose/g) | Adrenal gland | 7.0±2.1 | 3.9±0.8 | 3.5±0.4 | 4.1±1.0 | 2.0±1.5 |
| | Pancreas | 3.4±0.3 | 2.7±0.2 | 2.6±0.2 | 2.4±0.4 | 1.7±0.2 |
| | Blood | 4.5±0.2 | 4.5±0.2 | 4.3±0.4 | 3.5±0.5 | 2.8±0.2 |
| | Heart | 4.5±0.6 | 3.8±0.2 | 3.7±0.5 | 2.9±0.5 | 2.4±0.4 |
| | Lung | 4.6±0.2 | 3.7±0.2 | 3.4±0.2 | 2.9±0.4 | 2.2±0.2 |
| | Stomach | 4.6±1.5 | 4.9±0.4 | 3.5±0.2 | 4.3±0.3 | 3.5±0.6 |
| | Small intestine | 5.6±0.3 | 5.3±0.6 | 5.0±0.9 | 3.7±0.7 | 2.4±0.1 |
| | Large intestine | 3.5±0.3 | 6.2±0.5 | 8.5±0.5 | 7.7±1.2 | 7.9±0.7 |
| | Liver | 7.5±0.5 | 6.0±0.4 | 5.7±0.6 | 5.0±0.3 | 4.7±0.6 |
| | Spleen | 3.6±0.2 | 3.1±0.2 | 3.1±0.1 | 2.3±0.6 | 2.0±0.2 |
| | Kidney | 4.4±0.5 | 3.5±0.6 | 3.6±0.4 | 3.0±0.3 | 2.8±0.4 |
| | Femur | 1.9±0.2 | 2.3±0.1 | 2.7±0.3 | 3.4±0.7 | 4.5±0.1 |
| | Adrenal glend/Blood ratio | 1.55±0.43 | 0.86±0.15 | 0.82±0.13 | 1.16±0.15 | 0.61±0.63 |
| | Adrenal gland/Liver ratio | 0.95±0.34 | 0.64±0.13 | 0.61±0.12 | 0.82±0.19 | 0.38±0.41 |
| | Adrenal gland/Kdney ratio | 1.60±0.50 | 1.11±0.15 | 0.98±0.15 | 1.40±0.34 | 0.58±0.54 |

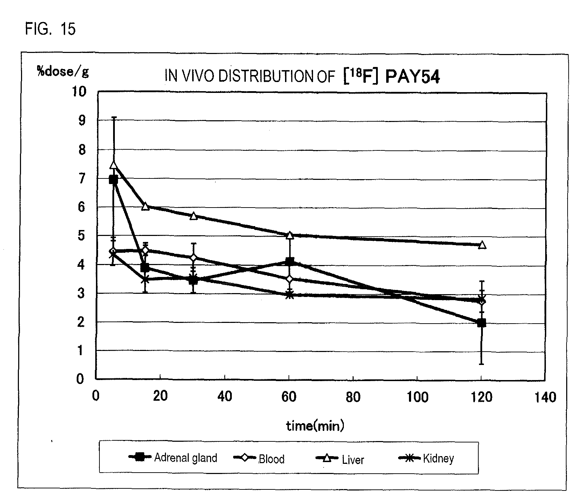

FIG. 15 shows time-dependent change in radioactivity accumulation in the adrenal gland, blood, liver, and kidney among the results shown in Table 5. 5 minutes after administration, high radioactivity accumulation was observed in the adrenal gland compared with blood and the kidney.

### Evaluation 5: Evaluation of affinity and selectivity

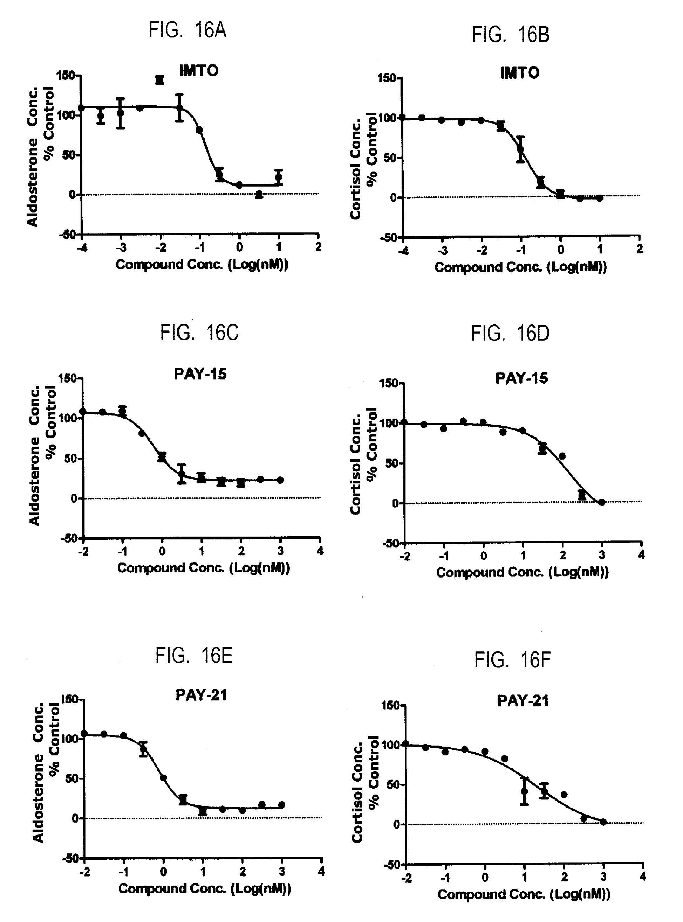

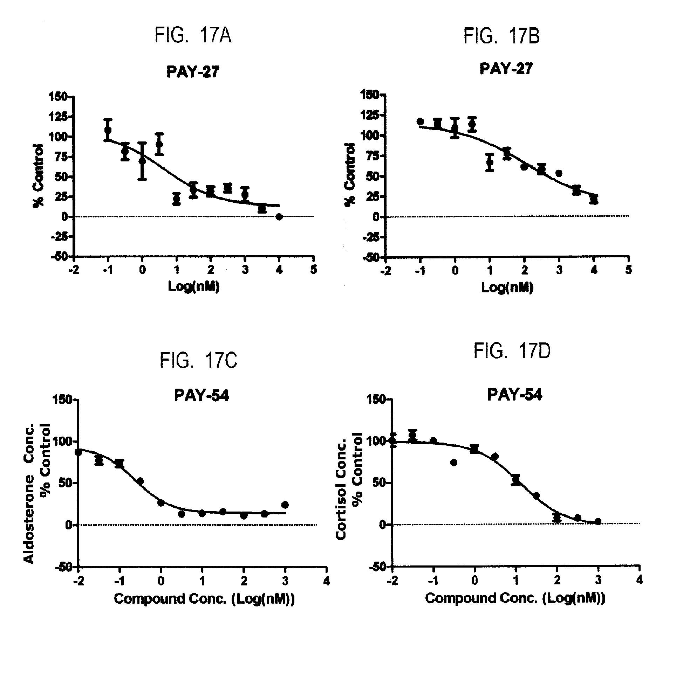

Chinese hamster lung-derived fibroblast V79 cells (obtained from European Collection of Cell Cultures (ECACC) via DS Pharma Biomedical Co., Ltd.) were allowed to express human CYP11B2 and human CYP11B1 to prepare V79-B2 and V79-B1, respectively. V79-B2 or V79-B1 was inoculated to a microplate and cultured overnight. Then, corticosterone and 11-deoxycortisol were added at final concentrations of 100 nmol/L into the culture supernatants of V79-B2 and V79-B1, respectively. At the same time, iodometomidate (IMTO) or the unlabeled form of PAY15, PAY21, PAY27, or PAY54 synthesized in Example 2, 5, 8, or 12 was added at a final concentration of 10⁻⁴ to 10³ nmol/L into the culture supernatants. One hour later, the culture supernatant of V79-B1 was recovered, and the concentration of cortisol, which is a metabolite of CYP11B1, was measured by enzyme-linked immunosorbent assay (ELISA). Four hours later, the culture supernatant of V79-B2 was recovered, and the concentration of aldosterone, which is a metabolite of CYP11B2, was measured by ELISA. As shown in FIGS. 16 and 17, an inhibition curve was prepared with aldosterone and cortisol concentrations in the absence of IMTO or PAY15, PAY21, PAY27, or PAY54 (unlabeled form) as 100% to calculate the inhibitory activity (IC₅₀) of each compound. FIG. 16A shows the inhibition curve of IMTO against CYP11B2. FIG. 16B shows the inhibition curve of IMTO against CYP11B1. FIG. 16C shows the inhibition curve of PAY15 against CYP11B2. FIG. 16D shows the inhibition curve of PAY15 against CYP11B1. FIG. 16E shows the inhibition curve of PAY21 against CYP11B2. FIG. 16F shows the inhibition curve of PAY21 against CYP11B1. FIG. 17A shows the inhibition curve of PAY27 against CYP11B2. FIG. 17B shows the inhibition curve of PAY27 against CYP11B1. FIG. 17C shows the inhibition curve of PAY54 against CYP11B2. FIG. 17D shows the inhibition curve of PAY54 against CYP11B1. IC₅₀ against cortisol production/IC₅₀ against aldosterone production was calculated and used as a selectivity factor for CYP11B2.

IC₅₀ of IMTO, PAY15, PAY21, PAY27, or PAY54 against aldosterone production and cortisol production as well as selectivity factors are shown in Table 6.

**Table 6**

| Compound | IC₅₀ against aldosterone production (nM) | IC₅₀ against cortisol production (nM) | **Selectivity factor** |
|---|---|---|---|
| **IMT0** | **0.14** | **0.13** | **0.93** |
| **PAY15** | **0.63** | **140.80** | **223.95** |
| **PAY21** | **0.81** | **21.06** | **26.09** |
| **PAY27** | **0.97** | **78.60** | **81.42** |
| **PAY54** | **0.24** | **13.13** | **55.49** |

PAY15, PAY21, PAY27, and PAY54 had a selectivity factor higher than that of IMTO described as a CYP11B imaging agent in Stefanie Hahner, et al., Journal of Clinical Endocrinology & Metabolism, 2008, Vol. 93, No. 6, p. 2358-2365 and as such, can serve as a more specific CYP11B2 imaging agent than IMTO. Stefanie Hahner, et al., Journal of Clinical Endocrinology & Metabolism, 2008, Vol. 93, No. 6, p. 2358-2365 has reported that the selectivity factor of IMTO is equivalent to other CYP11B imaging agents metomidate (MTO), etomidate (ETO), and fluoroetomidate (FETO) (IMTO, MTO, ETO, and FETO have selectivity factors of 0.261, 0.275, 0.208, and 0.145, respectively). These results demonstrated that PAY15, PAY21, PAY27, or PAY54 is highly specific for CYP11B2, compared with the known CYP11B imaging agents.

It is apparent that the present invention is not limited to the above embodiment, and may be modified and changed without departing from the scope and spirit of the invention.

It is intended to provide a radioactive compound that has higher selectivity for CYP11B2 than that for CYP11B1, exhibits highly selective accumulation in the adrenal gland compared with blood and organs adjacent to the adrenal gland, and permits commercial supply. The present invention provides a radioactive quinolinone derivative represented by the predetermined general formula or a salt thereof.

## Claims

1. A radioactive compound represented by the following general formula (1) or a salt thereof: 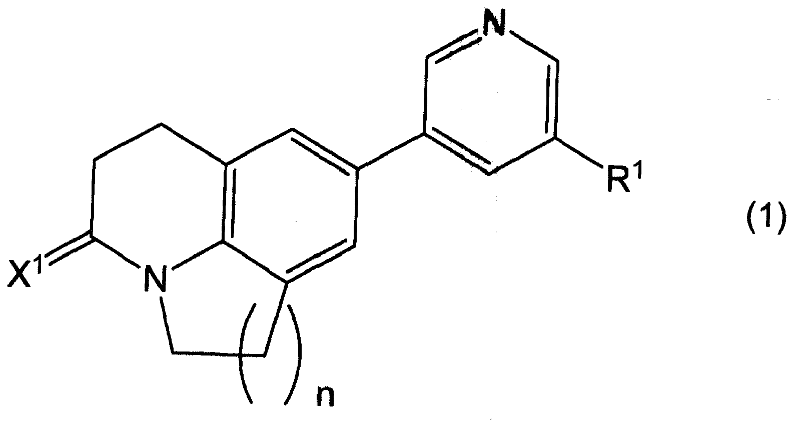 wherein R¹ represents -X² or -O(CH₂)ₘ-X²; X¹ represents an oxygen atom or a sulfur atom; X² represents a radiohalogen atom; n represents an integer of 1 or 2; and m represents an integer of 1 to 3.

2. The radioactive compound according to claim 1 or a salt thereof, wherein the radiohalogen atom is ¹⁸F, ¹²³I, ¹²⁴I, ¹²⁵I, ¹³¹I, or ⁷⁶Br in the general formula (1).

3. The radioactive compound according to claim 1 or 2 or a salt thereof, wherein X¹ represents an oxygen atom in the general formula (1).

4. The radioactive compound according to any one of claims 1 to 3 or a salt thereof, wherein R¹ represents a radioiodine atom, and X¹ represents an oxygen atom in the general formula (1).

5. The radioactive compound according to any one of claims 1 to 3 or a salt thereof, wherein R¹ represents - O(CH₂)ₘ-X², X¹ represents an oxygen atom, and X² represents a radiofluorine atom in the general formula (1).

6. A pharmaceutical drug comprising a radioactive compound according to any one of claims 1 to 5 or a salt thereof.

7. The pharmaceutical drug according to claim 6, wherein the pharmaceutical drug is a diagnostic imaging agent.

8. The pharmaceutical drug according to claim 6 or 7, wherein the pharmaceutical drug is a diagnostic imaging agent for adrenal disease.

9. The pharmaceutical drug according any one of claims 6 to 8, wherein the pharmaceutical drug is a diagnostic imaging agent for positron emission tomography.

10. The pharmaceutical drug according any one of claims 6 to 8, wherein the pharmaceutical drug is a diagnostic imaging agent for single-photon emission computed tomography.

11. A method for producing a radioactive compound represented by the following general formula (2) or a salt thereof through radioiodination reaction from a compound represented by the following general formula (6) or a salt thereof: 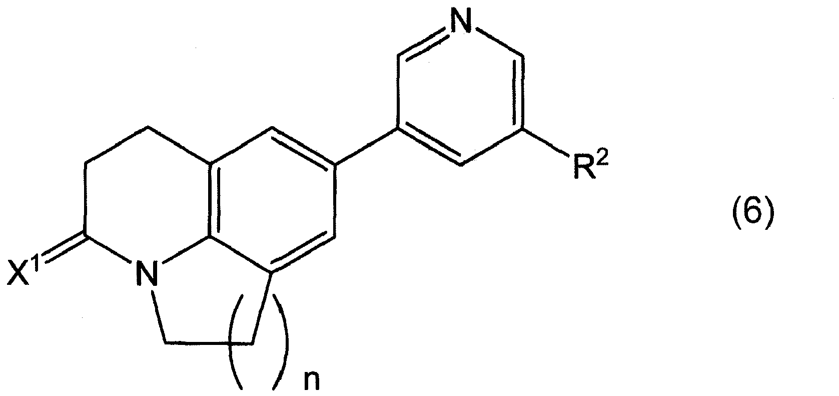 wherein X¹ represents an oxygen atom or a sulfur atom; R² represents a trialkyltin group or a trialkylsilyl group; and n represents an integer of 1 or 2, and 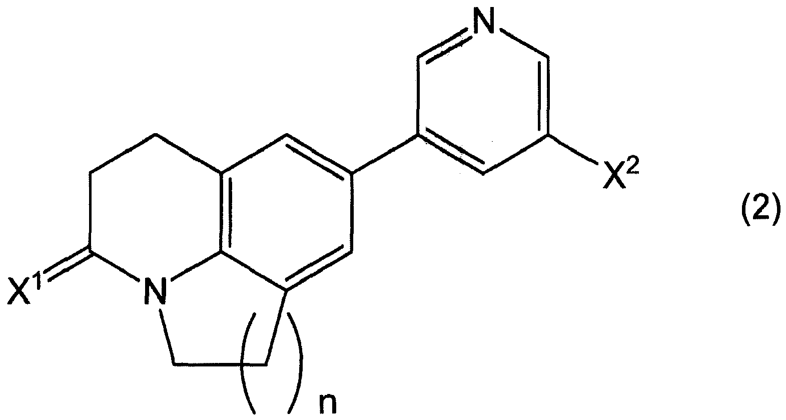 wherein X¹ represents an oxygen atom or a sulfur atom; X² represents a radioiodine atom; and n represents an integer of 1 or 2.

12. A method for producing a radioactive compound represented by the following general formula (3) or a salt thereof through radiofluorination reaction from a compound represented by the following general formula (8) or a salt thereof: 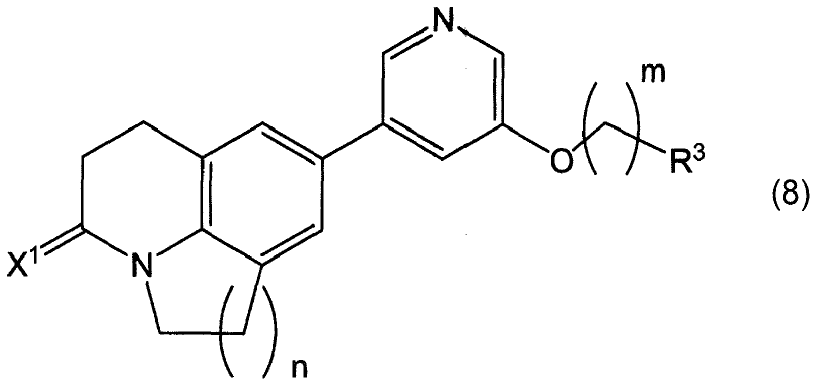 wherein X¹ represents an oxygen atom or a sulfur atom; R³ represents a halogen atom, a substituted or unsubstituted alkylsulfonyloxy group, or a substituted or unsubstituted arylsulfonyloxy group; n represents an integer of 1 or 2; and m represents an integer of 1 to 3, and 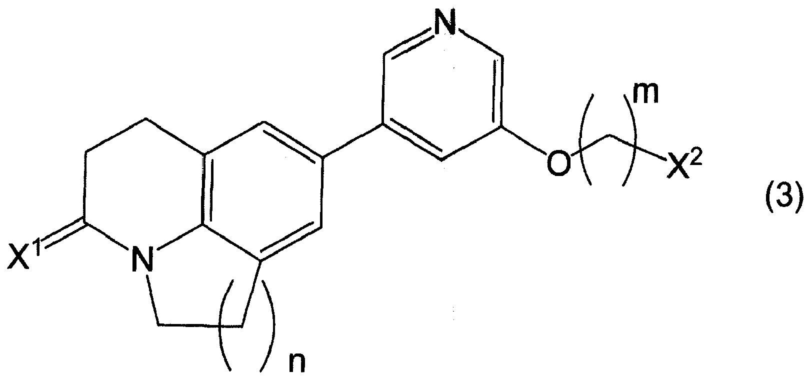 wherein X¹ represents an oxygen atom or a sulfur atom; X² represents a radiofluorine atom; n represents an integer of 1 or 2; and m represents an integer of 1 to 3.
